(19) Europäisches Patentamt
European Patent Office
Office européen des brevets

(11) Publication number: **0 074 268**
**A2**

(12) # EUROPEAN PATENT APPLICATION

(21) Application number: 82304674.3

(22) Date of filing: 07.09.82

(51) Int. Cl.[3]: C 07 D 501/46
A 61 K 31/545
//C07D471/04

(30) Priority: 08.09.81 US 300357
19.01.82 US 340628

(43) Date of publication of application:
16.03.83 Bulletin 83/11

(84) Designated Contracting States:
AT BE CH DE FR GB IT LI LU NL SE

(71) Applicant: ELI LILLY AND COMPANY
307, East McCarty Street
Indianapolis Indiana 46285(US)

(72) Inventor: Lunn, William Henry Walker
1141 East 80th Street
Indianapolis Indiana 46240(US)

(72) Inventor: Vasileff, Robert Theodore
4651 Cherry Lane
Indianapolis Indiana 46208(US)

(74) Representative: Crowther, Terence Roger et al,
Erl Wood Manor
Windlesham Surrey GU20 6PH(GB)

(54) **Improvements in or relating to thieno and furopyridinium-substituted cephalosporin derivatives.**

(57) Cephalosporin derivatives of formula (I):

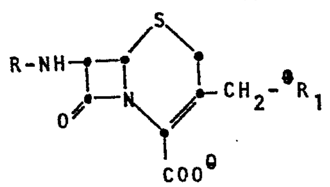

(1)

wherein R is an acyl group of the formula:

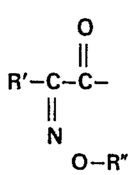

wherein R' is a 5- or 6-membered heterocyclic ring and ⊕$R_1$ is an optionally substituted thieno- or furo-pyridinium system are particularly effective antibacterial agents; or (R is hydrogen) useful intermediates.

Also disclosed is the useful and novel intermediate 2-carboxy-thieno(3,2-c)pyridine".

EP 0 074 268 A2

Croydon Printing Company Ltd.

# IMPROVEMENTS IN OR RELATING TO THIENO AND FUROPYRIDINIUM-SUBSTITUTED CEPHALOSPORIN DERIVATIVES

This invention relates to novel cephalosporin derivatives, more particularly to quaternary ammonium salts thereof having at the 3'-position a bicyclic thieno- or furopyridinium substituent.

A number of cephalosporin antibiotics substituted in the 3'-position by quaternary ammonium groups have been previously described in the literature. Such compounds possess the betaine structure in that the positively charged nitrogen atom of the quaternary ammonium group exists in the salt form with the anionic form of the $C_4$ carboxy group (carboxylate anion) of the cephalosporin system. For instance, the well-known cephalosporin antibiotic cephaloridine, 7-(α-thienylacetamido)-3-(pyridinium-1-ylmethyl)-3-cephem-4-carboxylate (see French Patent Specification No. 1,384,197 and Chemical Abstracts, 63, 1159lc (1965)) possesses this betaine structure.

Whilst, in general, existing cephalosporin quaternary ammonium salts possess good activity, in view of the known propensity of bacteria to mutate to provide resistant strains, there is a continuing need to develop new antibiotics. Further, not all of the quaternary ammonium salts developed have been free from untoward side-effects.

In accordance with the invention, it has now been discovered that compounds of formula (I):

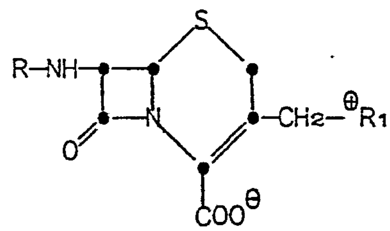 (I)

wherein R is hydrogen or an acyl group represented by the formula:

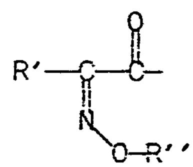

wherein R' is a 5- or 6-membered heterocyclic ring represented by the formulae:

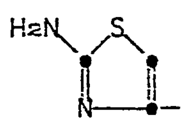 , 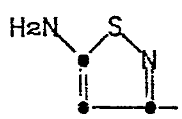 , 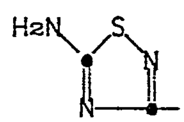 , 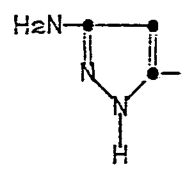 ,

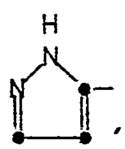 , 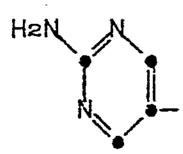 , 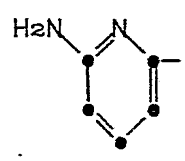 , or 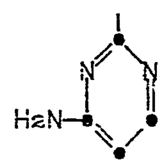 ;

R'' is hydrogen, $C_1$-$C_4$ alkyl, a carboxy-substituted alkyl or carboxy-substituted cycloalkyl group represented by the formula

$$-\overset{a}{\underset{b}{C}}-(CH_2)_n-COR'''$$

wherein n is 0-3, a and b when taken separately are independently hydrogen or $C_1$-$C_3$ alkyl, or when taken together with the carbon to which they are attached form a $C_3$-$C_7$ carbocyclic ring; R''' is hydroxy, amino, $C_1$-$C_4$ alkoxy, or OR° wherein R° is a carboxy-protecting ester group;

or R'' is a carbamoyl group represented by the formula

$$-\overset{O}{\overset{\|}{C}}-NHR''''$$

wherein R'''' is $C_1$-$C_4$ alkyl, phenyl, or $C_1$-$C_3$ alkyl substituted by phenyl; $^{\oplus}R_1$ is a bicyclic-pyridinium group selected from the group consisting of a thienopyridinium group represented by the formulae

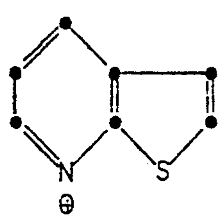

,

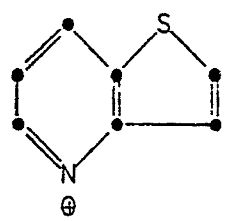

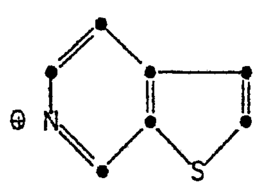

,

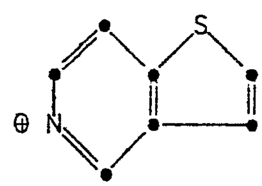

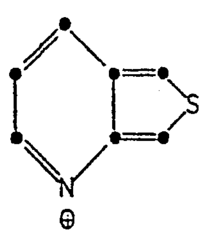

,

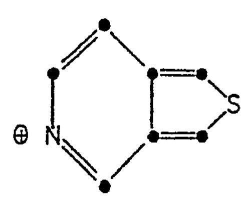

or a furopyridinium group represented by the formulae

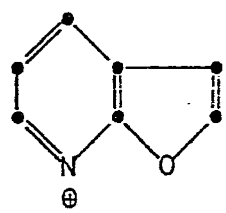 , 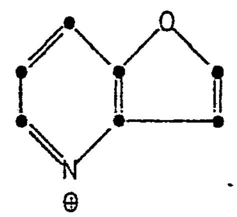 .

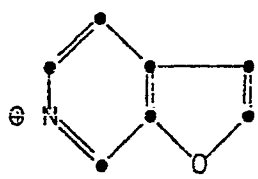 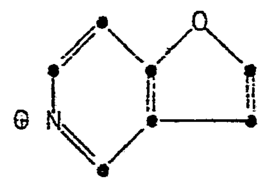 ,

where in the above formulae either or both of the hetero rings is optionally substituted by one or two substituents independently selected from $C_1$-$C_4$ alkyl, halogen, trifluoromethyl, carboxy, carbamoyl, $-SO_3H$, hydroxy, $C_1$-$C_4$ alkoxy, amino, mono $C_1$-$C_4$ alkylamino, di($C_1$-$C_4$ alkyl) amino, $C_{2-4}$ alkanoylamino, aminosulphonyl, cyano, $C_1$-$C_4$ alkoxycarbonyl or a group of formula

$$-\overset{O}{\overset{\|}{C}}-NR_5-OR_6$$

where $R_5$ and $R_6$ are independently hydrogen or $C_1$-$C_4$ alkyl; or pharmaceutically-acceptable salts thereof; are particularly effective in combatting bacterial infections, :: (R is hydrogen) are useful intermediates.

The terms used in the definition of the compounds of the formula (I) have the following meanings herein: "$C_1$-$C_4$ alkyl" refers to methyl, ethyl, n-propyl, isopropyl, n-butyl, t-butyl, sec-butyl, and the like; "$C_1$-$C_4$ alkoxy" refers to methoxy, ethoxy, n-propoxy, isopropoxy, n-butoxy, t-butoxy, sec-butoxy, and the like; "$C_1$-$C_3$ alkyl" refers to methyl, ethyl, n-propyl, and isopropyl; "$C_1$-$C_3$ alkyl substituted by phenyl" refers to benzyl, 2-phenethyl, 1-phenethyl, 3-phenylpropyl, 2-phenylpropyl, and the like; and "$C_3$-$C_7$ carbocyclic ring" refers to cyclopropyl, cyclobutyl, cyclopentyl, cyclohexyl, and cycloheptyl.

Halogen preferably refers to fluoro, chloro or bromo.

The carboxy-substituted alkyl and carboxy-substituted cycloalkyl groups represented by R'' in formula (I) when R''' is hydroxy are exemplified by carboxymethyl, 2-carboxyethyl, 3-carboxypropyl, 4-carboxybutyl, 2-carboxyprop-2-yl, 2-carboxyprop-1-yl, 2-methyl-4-carboxybut-2-yl, 3-carboxy-3-methylprop-2-yl, 1-carboxycycloprop-1-yl, 1-carboxycyclobut-1-yl, 1-carboxycyclopent-1-yl, 1-carboxycyclohex-1-yl, 1-carboxymethylcyclobut-1-yl, 2-carboxyethylcyclohex-1-yl, and the like. When in the formula (I), R''' is $NH_2$, examples of the carboxamides represented are the amides of the above-named carboxy-substituted radicals.

The esters of the carboxy-substituted groups (formula (I), R'' is carboxy-substituted alkyl or cycloalkyl and R''' is $C_1$-$C_4$ alkoxy) are illustrated by methoxycarbonylmethyl, ethoxycarbonylmethyl, 2-(ethoxycarbonyl)prop-2-yl, 1-propoxycarbonylcyclopent-1-yl, and like $C_1$-$C_4$ alkyl esters of the above-named carboxy-substituted alkyl and cycloalkyl radicals.

Examples of N-substituted carbamoyl groups (formula (I), R'' is carbamoyl) are N-methylcarbamoyl,

N-ethylcarbamoyl, N-propylcarbamoyl, N-phenylcarbamoyl, N-benzylcarbamoyl, and the like.

The terms "thienopyridinium" and "furopyridinum", represented by $^{\oplus}R_1$ in formula (I), refer to bicyclic thieno- and furopyridine systems bonded to the 3-position methylene group of the cephalosporin dihydrothiazine ring via a positively-charged nitrogen atom in the pyridine moiety. As described herein, the compounds of the invention are betaines wherein the quaternary ammonium group exists with an anion, commonly, the $C_4$ carboxylate anion as depicted by formula (I).

The heterocyclic rings represented by R' in formula (I) are described herein using the following nomenclature: 2-aminothiazol-4-yl, 5-aminoisothiazol-3-yl, 5-amino-1,2,4-thiadiazol-3-yl, pyrazol-5-yl, 3-aminopyrazol-5-yl, 2-aminopyrimidin-5-yl, 4-aminopyrimidin-2-yl and 2-aminopyridin-6-yl.

In the description of the compounds of the invention, the term "oximino" will be used for convenience in describing the oxime and substituted oxime function:

$$\begin{array}{c}-C-\\ \Vert\\ N-O-R''\end{array}\quad .$$

One class of compounds of formula (I) which may be mentioned is that wherein either or both of the thieno or furopyridinium rings is optionally substituted by one or two $C_1$-$C_4$ alkyl, fluoro, chloro, bromo, carboxy, carbamoyl or $C_1$-$C_4$ alkoxycarbonyl groups.

A further class of compounds of formula (I) worthy of mention is that where $^{(+)}R_1$ is an unsubstituted thienopyridinium system.

Preferred compounds in accordance with the invention may have one or more of the following features:

(A) R' is 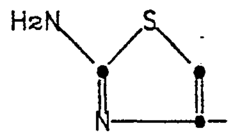

(B) R'' is methyl;

(C) the thieno or furopyridinium system is monosubstituted;

(D) the thieno or furopyridinium system is monosubstituted with $C_1$-$C_4$ alkyl;

(E) the thieno or furopyridinium system is monosubstituted with halogen;

(F) the thieno or furopyridinium system is monosubstituted with carboxy;

(G) the thieno or furopyridinium system is monosubstituted with carbamoyl;

(H) the thieno or furopyridinium system is monosubstituted with $-SO_3H$;

(I) the thieno or furopyridinium system is monosubstituted with $C_1$-$C_4$ alkoxy;

(J) the thieno or furopyridinium system is monosubstituted with amino;

(K) the thieno or furopyridinium system is monosubstituted with $C_{2-4}$ alkanoylamino;

(L) the thieno or furopyridinium system is monosubstituted with aminosulphonyl;

(M) the thieno or furopyridinium system is monosubstituted with $C_1$-$C_4$ alkoxycarbonyl; and

(N) the thieno or furopyridinium system is monosubstituted with a group of formula:

$$-\overset{\displaystyle O}{\overset{\|}{C}}-NR_5-OR_6$$

where $R_5$ and $R_6$ are independently hydrogen or $C_1$-$C_4$ alkyl.

Preferred compounds of the invention possess the 7-amino side chain:

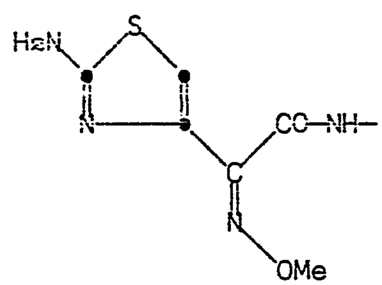

in which the oxime has a syn-configuration.
The presently preferred compound of the invention is syn-7-[2-(2-aminothiazol-4-yl)-2-methoxyiminoacetamido]-3-(2-carboxythieno[3,2-c]pyridinium-5-ylmethyl)-3-cephem-4-carboxylate.
Preferred compounds of the invention, as well as exhibiting a high level of antibacterial activity against a broad spectrum of bacterial microorganisms, for instance being highly effective against gram-negative bacteria, also are free from untoward side-effects, for instance exhibiting little or no nephrotoxicity in in vitro tests.

Examples of bicyclicpyridinium compounds of the invention represented by the formula (I) include the following compounds:

7-[2-(2-aminothiazol-4-yl)-2-hydroxyimino-acetamido]-3-(thieno[2,3-b]pyridinium-7-ylmethyl)-3-cephem-4-carboxylate,

7-[2-(2-aminothiazole-4-yl)-2-methoxyimino-acetamido]-3-(thieno[2,3-b]pyridinium-7-ylmethyl)-3-cephem-4-carboxylate,

7-[2-(2-aminothiazole-4-yl)-2-ethoxycarbonyl-methoxyiminoacetamido]-3-(thieno[2,3-b]pyridinium-7-ylmethyl)-3-cephem-4-carboxylate,

7-[2-(2-aminothiazole-4-yl)-2-methoxyimino-acetamido]-3-(thieno[3,2-b]pyridinium-4-ylmethyl)-3-cephem-4-carboxylate,

7-[2-(2-aminothiazol-4-yl)-2-methoxyimino-acetamido]-3-(thieno[2,3-c]pyridinium-6-ylmethyl)-3-cephem-4-carboxylate,

7-[2-(2-aminothiazol-4-yl)-2-methoxyimino-acetamido]-3-(thieno[3,2-c]pyridinium-5-ylmethyl)-3-cephem-4-carboxylate,

7-[2-(2-aminothiazol-4-yl)-2-methoxyimino-acetamido]-3-(thieno[3,4-b]pyridinium-4-ylmethyl)-3-cephem-4-carboxylate,

7-[2-(2-aminothiazol-4-yl)-2-methoxyimino-acetamido]-3-(thieno[3,4-c]pyridinium-5-ylmethyl)-3-cephem-4-carboxylate,

7-[2-(2-aminopyridin-6-yl)-2-(2-carboxypropyl)oxyiminoacetamido)-3-(thieno[3,4-b]pyridinium-4-ylmethyl)-3-cephem-4-carboxylate,

7-[2-(5-amino-1,2,4-thiadiazol-3-yl)-2-methoxyiminoacetamido]-3-(thieno[2,3-b]pyridinium-7-ylmethyl)-3-cephem-4-carboxylate,

7-[2-(5-aminoisothiazol-3-yl)-2-(2-carboxyprop-2-yl)oxyiminoacetamido]-3-(thieno[2,3-c]pyridinium-6-ylmethyl)-3-cephem-4-carboxylate,

7-[2-(2-aminopyrimidin-5-yl)-2-ethoxyiminoacetamido]-3-(thieno[3,2-b]pyridinium-4-ylmethyl)-3-cephem-4-carboxylate,

7-[2-(4-aminopyrimidin-2-yl)-2-(N-methylcarbamoyloxy)iminoacetamido]-3-(thieno[3,2-c]pyridinium-5-ylmethyl)-3-cephem-4-carboxylate,

7-[2-(2-aminopyridin-6-yl)-2-methoxyiminoacetamido]-3-(thieno[3,2-b]pyridinium-4-ylmethyl)-3-cephem-4-carboxylate,

7-[2-(3-aminopyrazol-5-yl)-2-methoxyiminoacetamido]-3-(thieno[2,3-b]pyridinium-7-ylmethyl)-3-cephem-4-carboxylate,

7-[2-(3-aminopyrazol-5-yl)-2-ethoxycarbonylmethoxyiminoacetamido]-3-(thieno[3,2-c]pyridinium-5-ylmethyl)-3-cephem-4-carboxylate,

7-[2-(pyrazol-5-yl)-2-methoxycarbonylmethoxyiminoacetamido]-3-(thieno[3,2-b]pyridinium-4-ylmethyl)-3-cephem-4-carboxylate,

7-[2-(pyrazol-5-yl)-2-ethoxycarbonylmethoxyiminoacetamido]-3-(thieno[2,3-c]pyridinium-6-ylmethyl)-3-cephem-4-carboxylate,

7-[2-(pyrazol-5-yl)-2-methoxyiminoacetamido]-3-(thieno[3,2-b]pyridinium-4-ylmethyl)-3-cephem-4-carboxylate,

7-[2-(2-aminothiazol-4-yl]-2-(2-carboxyprop-2-yl)oxyiminoacetamido]-3-(thieno[3,2-c]pyridinium-5-ylmethyl)-3-cephem-4-carboxylate,

7-[2-(2-aminothiazol-4-yl)-2-(2-carboxyprop-2-yl)oxyiminoacetamido]-3-(thieno[3,2-b]pyridinium-4-ylmethyl)-3-cephem-4-carboxylate,

7-[2-(2-aminothiazol-4-yl)-2-(2-carboxyprop-2-yl)oxyiminoacetamido]-3-(thieno[2,3-c]pyridinium-6-ylmethyl)-3-cephem-4-carboxylate,

7-[2-(2-aminothiazol-4-yl)-2-(2-carboxyprop-2-yl)oxyiminoacetamido]-3-(thieno[3,4-b]pyridinium-7-ylmethyl)-3-cephem-4-carboxylate,

7-[2-(2-aminopyridin-6-yl)-2-methoxyiminoacetamido]-3-(thieno[2,3-c]pyridinium-6-ylmethyl)-3-cephem-4-carboxylate,

7-[2-(2-aminopyridin-6-yl)-2-methoxyiminoacetamido]-3-(thieno[3,2-c]pyridinium-5-ylmethyl)-3-cephem-4-carboxylate,

7-[2-(2-aminopyridin-6-yl)-2-ethoxycarbonylmethoxyiminoacetamido]-3-(thieno[2,3-c]pyridinium-6-ylmethyl)-3-cephem-4-carboxylate, and

7-[2-(5-amino-1,2,4-thiadiazol-3-yl)-2-oximinoacetamido]-3-(thieno[2,3-b]pyridinium-7-ylmethyl)-3-cephem-4-carboxylate,

7-[2-(2-aminothiazol-4-yl)-2-methoxyiminoacetamido]-3-(4-methylthieno[2,3-b]pyridinium-7-ylmethyl)-3-cephem-4-carboxylate,

7-[2-(2-aminothiazol-4-yl)-2-methoxyiminoacetamido]-3-(7-methylthieno[3,2-b]pyridinium-4-ylmethyl)-3-cephem-4-carboxylate,

7-[2-(2-aminothiazol-4-yl)-2-methoxyiminoacetamido]-3-(2-ethylthieno[2,3-b]pyridinium-7-ylmethyl)-3-cephem-4-carboxylate,

7-[2-(2-aminothiazol-4-yl)-2-ethoxyimino-acetamido]-3-(2,3-dibromothieno[2,3-b]pyridinium-7-ylmethyl)-3-cephem-4-carboxylate,

7-[2-(pyrazol-5-yl)-2-ethoxycarbonylmethoxy-iminoacetamido]-3-(7-methylthieno[3,2-c]pyridinium-4-ylmethyl)-3-cephem-4-carboxylate,

7-[2-(2-aminothiazol-4-yl)-2-methoxyimino-acetamido]-3-(furo[2,3-c]pyridinium-6-ylmethyl)-3-cephem-4-carboxylate,

7-[2-(2-aminothiazol-4-yl)-2-methoxyimino-acetamido]-3-(furo[3,2-c]pyridinium-5-ylmethyl)-3-cephem-4-carboxylate,

7-[2-(2-aminothiazol-4-yl)-2-methoxyimino-acetamido]-3-(furo[2,3-b]pyridinium-7-ylmethyl)-3-cephem-4-carboxylate,

7-[2-(2-aminothiazol-4-yl)-2-methoxyimino-acetamido]-3-(2-methylfuro[3,2-c]pyridinium-5-ylmethyl)-3-cephem-4-carboxylate,

7-[2-(2-aminothiazol-4-yl)-2-methoxyimino-acetamido]-3-(2,7-dimethylfuro[3,2-c]pyridinium-5-ylmethyl)-3-cephem-4-carboxylate,

7-[2-(2-aminothiazol-4-yl)-2-(2-carboxyprop-2-yl)oxyiminoacetamido]-3-(furo[3,2-c]pyridinium-5-ylmethyl)-3-cephem-4-carboxylate,

7-[2-(5-amino-1,2,4-thiadiazol-3-yl)-2-methoxyiminoacetamido]-3-(furo[3,2-c]pyridinium-5-ylmethyl)-3-cephem-4-carboxylate,

7-[2-(pyrazol-5-yl)-2-ethoxycarbonylmethoxy-iminoacetamido]-3-[furo[3,2-c]pyridinium-5-ylmethyl)-3-cephem-4-carboxylate,

7-[2-(2-aminothiazol-4-yl)-2-methoxyimino-

acetamido]-3-(furo[3,2-b]pyridinium-4-ylmethyl)-3-cephem-4-carboxylate,

7-[2-(2-aminothiazol-4-yl)-2-(2-carboxyprop-2-yl)oxyiminoacetamido]-3-(furo[3,2-b]pyridinium-4-ylmethyl)-3-cephem-4-carboxylate,

7-[2-(2-aminopyrimidin-5-yl)-2-ethoxyiminoacetamido]-3-(2-methylfuro[3,2-b]pyridinium-4-ylmethyl)-3-cephem-4-carboxylate,

7-[2-(2-aminothiazol-4-yl)-2-hydroxyiminoacetamido]-3-(furo[3,2-c]pyridinium-5-ylmethyl)-3-cephem-4-carboxylate,

7-[2-(3-aminopyrazol-5-yl)-2-methoxyiminoacetamido]-3-(furo[2,3-b]pyridinium-7-ylmethyl)-3-cephem-4-carboxylate,

7-[2-(2-aminothiazol-4-yl)-2-(2-carboxyprop-2-yl)oxyiminoacetamido]-3-(2,4-dimethylfuro[2,3-b]-pyridinium-7-ylmethyl)-3-cephem-4-carboxylate, and

7-[2-(5-amino-1,2,4-thiadiazol-3-yl)-2-methoxyiminoacetamido]-3-(furo[2,3-b]pyridinium-7-ylmethyl)-3-cephem-4-carboxylate.

The thienopyridines which can be used to form the compounds of the invention are represented by the following structural formulae wherein the numbering system indicated is employed in naming compounds of the invention herein.

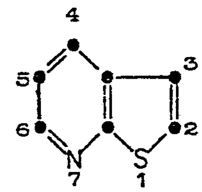

Thieno[2,3-b]pyridine

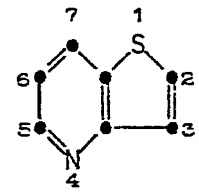

Thieno[3,2-b]pyridine

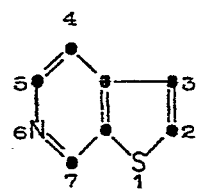

Thieno[2,3-c]pyridine

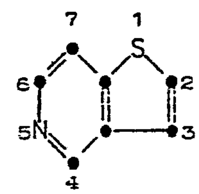

Thieno[3,2-c]pyridine

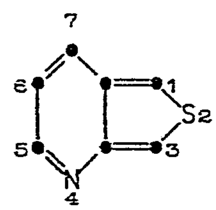

Thieno[3,4-b]pyridine

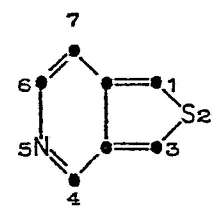

Thieno[3,4-c]pyridine

The thienopyridines are known or can be prepared by literature procedures. Klemm, et al., J. Org. Chem., 34, [2] 347-353 (1969) describe the preparation of thieno[2,3-b] and [3,2-b]pyridines; the thieno[3,4-b] and [3,4-c]pyridines are described by Klemm, et al., J. Heterocyclic Chem., 9, 843 (1972); the thieno[2,3-c]pyridine is described by Klemm, et al., J. Heterocyclic Chem., 5, 883 (1969), and the thieno[3,2-c]pyridine by S. Gronowitz and E. Sandberg, Arkiv Kemi, 32, 217 (1970), and Eloy, et al., Bull. Soc. Chim. Belges, 79, 301 (1970). The thieno[2,3-c] and [3,2-c]-pyridines also are described by J. P. Maffrand and F. Eloy, J. Heterocyclic Chem., 13, 1347 (1976) and Heterocycles, 12, No. 11 (1979).

The furopyridines used in the preparation of the compounds of the invention are also known or can be prepared by known procedures. In the following formulae representing these furopyridines, the numbering system designated is used in naming the compounds of the invention.

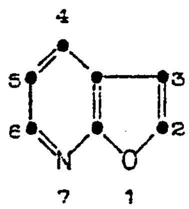

Furo[2,3-b]pyridine

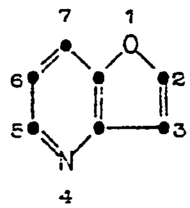

Furo[3,2-b]pyridine

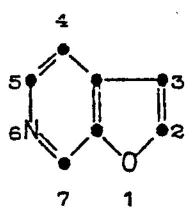

Furo[2,3-c]pyridine

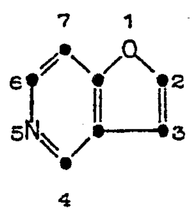

Furo[3,2-c]pyridine

The furo[3,2-c]pyridines can be prepared as described by F. Eloy, et al., J. Het. Chem. 8, 57-60, 1971. The furo[2,3-b]pyridines and the furo[3,2-b]-pyridines are prepared by the methods described by J. W. McFarland, et al., J. Heterocyclic Chem., 8, 735-738 (1971) and S. Gronowitz, et al., Acta Chemica Scandinavica, B-29, 233-238 (1975), respectively.

Furo[2,3-c]pyridine can be prepared by methods akin to those employed in the preparation of the corresponding thieno[2,3-c]pyridine by using furan-3-aldehyde which can be converted to furan-3-acrylic acid and the acid converted to the acid azide by first reacting the acid with ethoxycarbonyl chloride and

triethylamine to form the acid chloride and then reacting the mixed anhydride with sodium azide in water. The furan-3-acrylic acid azide can then be heated in diphenylmethane with tributylamine to a temperature of about 190°C. to about 210°C. to form the furopyridone represented by the formula

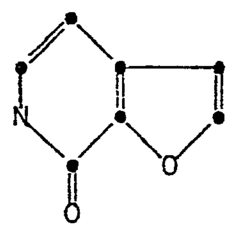

The furopyridone may be reacted with phosphorus oxychloride to form 7-chlorofuro[2,3-c]pyridine and the latter reductively dechlorinated with zinc in acetic acid to provide furo[2,3-c]pyridine.

Carboxy-protected derivatives of the compounds represented by the formula (I) when R'' is a carboxy-substituted alkyl or carboxy-substituted cycloalkyl group and R''' is OR°, are esters of the carboxy group commonly known in the art as carboxy-protecting or blocking groups. Examples of such ester groups (R°) are alkyl, alkenyl, and substituted alkyl ester groups such as t-butyl, 2-methylbutene-2-yl, 2,2,2-trichloroethyl, 2,2,2-tribromoethyl, and 2-iodoethyl; the benzyl ester and substituted benzyl esters such as p-methoxybenzyl and p-nitrobenzyl; the diphenylmethyl ester and substituted diphenylmethyl esters such as the 4-methoxydiphenylmethyl and 4,4'-dimethoxydiphenylmethyl esters; and trialkylsilyl esters, e.g., tri $C_1$-$C_4$ alkylsilyl esters such as trimethylsilyl; and like ester groups. The carboxy-protecting group is used for the temporary protection of the carboxy group as, for example, during the preparation of the compounds. These groups are readily removed under hydrolysis or hydrogenolytic conditions generally known in the art.

The compounds of the invention wherein R is an acyl group of the formula

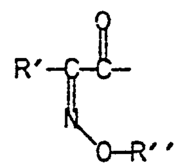

are broad spectrum antibiotics which inhibit the growth of microorganisms pathogenic to man and animals. For example, these compounds are effective in controlling the growth of the staphylococci and streptococci and penicillin-resistant strains of staphylococci. They also inhibit the growth of the gram-negative bacteria for example, proteus, pseudomonas, enterobacter, Escherichia coli, klebsiella, shigella, serratia, and salmonella.

Compounds represented by the formula (I) in which R is hydrogen, are valuable intermediates as will be described hereinafter.

In a further aspect of the invention there is provided a process for preparing a compound of formula (I) which comprises:

(a) condensing a compound of formula:

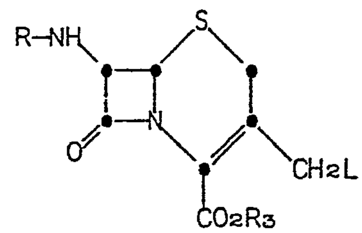

(II)

where L is a leaving group and $R_3$ is hydrogen or an ester protecting group; with a thieno or furopyridine of formula $R_1$, followed, in the case where $R_3$ is an ester protecting group, by removal of that group and any protecting groups which may be present in the R-substituent;

(b) acylating a compound of formula

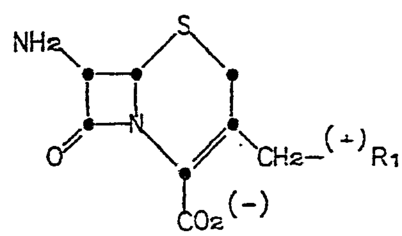

(III)

, where $R_1$ is as defined above, or a salt or 4'-ester ($R_3$) thereof, with an active carboxy derivative of an acid of formula

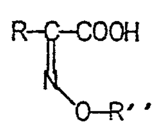

followed, in the case where the compound of formula (III) is in the form of an ester by removal of the $R_3$ group, together with any amino protecting groups which may be present in R-substituent.

As stated previously, the preferred compounds of the invention are those possessing the 7-acyamino side chain

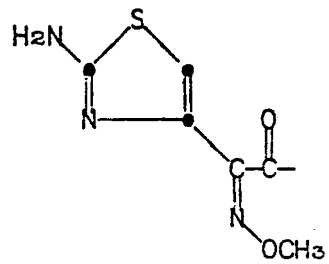

In such a situation, the process of the invention would involve either

(a) condensing a compound of formula:

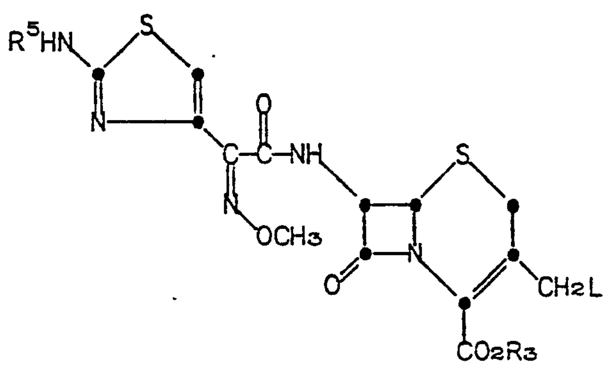

where $R^5$ is hydrogen or an amino-protecting group, with a thieno or furopyridine of formula $R_1$, followed by removal of $R^5$ or $R^3$, if either or both of them are protecting groups, or

(b) acylating a compound of formula (III), or ester or salt thereof, with an active carboxy derivative of formula

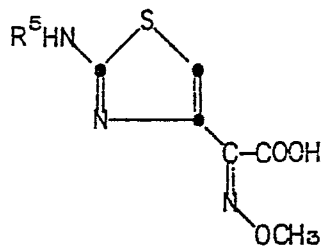

followed by removal of any protecting groups present in the final product.

The nature of blocking groups suitable for protecting the 4'-acid function will be well-known to those skilled in the art, see for example, the previous commentary concerning the R° groups. The use of silyl protecting groups such as tri $C_1$-$C_4$ alkylsilyl protecting groups is preferred. Amino-protecting groups ($R^5$) which can be used are those commonly employed in the cephalosporin art for the temporary protection of basic amino groups to block or prevent their interference in a reaction carried out at another site in the molecule. Examples of such groups are the haloacyl groups such as chloroacetyl and dichloroacetyl; the urethane-forming protecting groups such as t-butyloxycarbonyl, 2,2,2-trichloroethoxycarbonyl, cyclopentyloxycarbonyl, adamantyloxycarbonyl, benzyloxycarbonyl, p-nitrobenzyloxycarbonyl, and diphenylmethyloxycarbonyl; and other protecting groups such as trityl (triphenylmethyl) and benzhydryl.

Both reactions (a) and (b) may be effected at temperatures within the range -20 to 100°C, preferably from 0 to 60°C, typically at room temperature.

Aprotic organic solvents such as acetonitrile or methylene chloride are suitable for carrying out reaction

(a), whereas the acylation reaction (b) may be carried out in aqueous media such as $H_2O$/acetonitrile, or in aprotic organic solvents such as tetrahydrofuran or acetonitrile.

As stated above, pharmacologically-active compounds of the invention can be prepared by the reaction of a thienopyridine or a furopyridine with a 7-acylaminocephalosporin represented by the formula (IV)

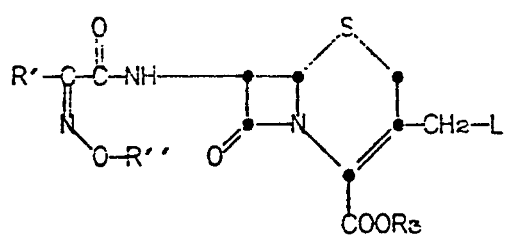

(IV)

wherein R', R'', $R_3$ and L are as defined above and L may be for example chloro, bromo, iodo or acetoxy, preferably iodo. In the preferred method, a compound of the formula (IV) wherein L is iodo is first prepared by the reaction of a compound of the formula (IV) wherein L is acetoxy and $R_3$ is an ester group with trimethylsilyliodide (trimethyliodosilane) by the method described in Bonjouklian's U.S. Patent No. 4,266,049. The 3-iodomethyl cephalosporin may then be reacted with the thienopyridine or furopyridine to provide a compound of the invention.

In carrying out the preferred process, a compound of the formula (IV) (L is acetoxy) is initially silylated to form the silyl ester of the $C_4$ carboxy group and with other silyl reactive groups. The

silylation can be carried out at room temperature in an aprotic organic solvent with a silylating reagent such as mono- or bis-trimethylsilylacetamide, mono-trimethylsilyltrifluoroacetamide, or N-methyl-N-trimethylsilyltrifluoroacetamide. The silylated derivative may then be reacted at ambient temperature with trimethylsilyliodide to provide the silylated 3-iodomethyl cephalosporin. The silylated 3-iodomethyl cephalosporin can then be reacted with the thienopyridine or the furopyridine to provide a silylated compound of the invention. Hydrolysis of the silyl groups provides a compound of the invention.

The process is illustrated by the following reaction scheme wherein a trimethyl silylating reagent and a thieno[2,3-b]pyridine are exemplified.

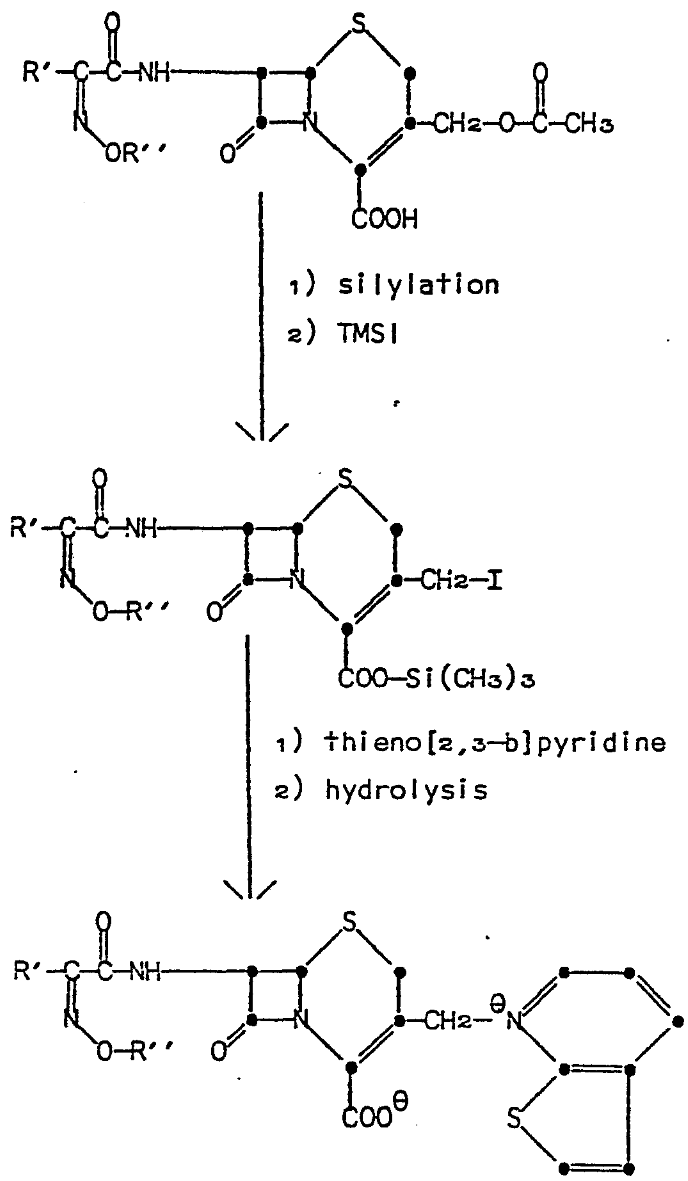


In the above scheme, R' and R'' have the same meanings as defined hereinabove.

If other $R_3$ ester protecting groups are utilised, these may be removed by hydrogenolysis or hydrolytic procedures.

Alternatively, the compounds of the invention can be prepared with a compound of the formula (IV) (L is acetoxy) by displacement of the acetoxy group with the thienopyridine or furopyridine. The preparation can be carried out in a known manner, for instance, in an aqueous medium, for example in a water miscible organic solvent containing water. The addition of a small amount of an alkali metal iodide such as potassium iodide can enhance the rate of the reaction. The reaction can be carried out at a temperature between about 35°C. and about 70°C. Water miscible organic solvents such as acetone, acetonitrile, tetrahydrofuran and dimethylacetamide may be mentioned as useful solvents.

The compounds of the formula (I), wherein R is hydrogen or formyl, can be prepared with 7-aminocephalosporanic acid and 7-formamidocephalosporanic acid, respectively, by displacement of the 3'-acetoxy group with the thienopyridine or furopyridine as described above. Alternatively, 7-formamido-3-iodomethyl-3-cephem-4-carboxylic acid trimethylsilyl ester can be prepared by the method described in U.S. Patent No. 4,266,049 and then be reacted with the appropriate thienopyridine or the furopyridine to provide a compound of the formula (I) wherein R is formyl.

Alternatively, the 7-amino nucleus compounds of formula (I) (R is H) can be prepared by the well-known N-deacylation reaction which proceeds through an imino chloride to an imino ether and thence on decomposition of the latter to the 7-amino-3-bicyclicpyridinium-4-carboxylate. Initially, a 7-acylaminocephalo-

sporanic acid, wherein the 7-acyl group can be for example phenylacetyl, phenoxyacetyl or a heterocyclic acyl group such as thienylacetyl, can be reacted with the bicyclicpyridine to form the 7-acylamino-3-bicyclicpyridinium-methyl)-3-cephem-4-carboxylate. Alternatively, the latter compound may be obtained via the 7-acylamino-3-iodomethyl ester which is allowed to react with the bicyclicpyridine. The 7-acyl bicyclopyridinium compound can then be treated with an imino halide-forming reagent such as phosphorus pentachloride in an inert solvent in the presence of an acid-binding agent such as a tertiary amine e.g., diethylaniline to provide the imino halide derivative of the 7-position acylamido group. Without isolation, the imino halide is treated with an alcohol, alkanediol or benzyl alcohol to form the corresponding imino ether. Decomposition of the imino ether provides the 7-amino nucleus compound.

In an example of the preparation of a 7-amino nucleus compound by this method, 7-(2-thienylacetamido)-cephalosporanic acid is reacted with thieno[2,3-b]pyridine to prepare 7-(2-thienylacetamido)-3-(thieno[2,3-b]pyridinium-7-ylmethyl)-3-cephem-4-carboxylate. The latter is converted to the trimethylsilyl ester with trimethylchlorosilane in a halogenated hydrocarbon solvent in the presence of dimethylacetamide (weak base) in an amount corresponding to a 4-5 molar excess. Solvents such as methylene chloride, trichloroethane, and chloroform are suitable. The solution of the silyl ester is cooled to a temperature of about -30°C. to about 0°C. and an imino halide-forming reagent such as

phosphorus pentachloride is added. After imino halide formation is complete, a $C_1$-$C_4$ alkanol, an alkanediol, or a benzyl alcohol is added to the cold reaction mixture. The temperature of the reaction mixture is allowed to warm to about room temperature and the product, 7-amino-3-(thieno[2,3-b]pyridinium-7-ylmethyl)-3-cephem-4-carboxylic acid, precipitates in the form of the dihydrochloride salt represented by the formula

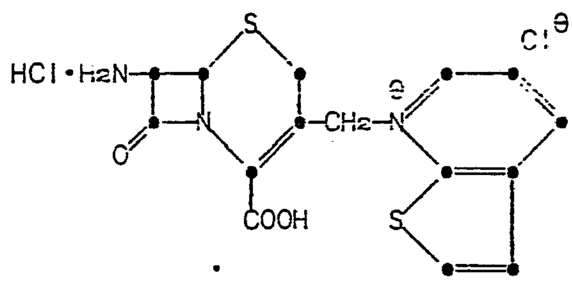

The N-formyl compounds (formula (I), R is formyl) are useful as intermediates in the preparation of the antibiotic compounds of the invention. For example, 7-formamidocephalosporanic acid can be silylated and the silyl ester converted to the 3-iodomethyl derivative with trimethylsilyliodide as described hereinabove. The 3-iodomethyl silylated derivative may then be reacted with the bicyclicpyridine to a compound in which R is formyl. The N-formyl-3-thienopyridinium-methyl-3-cephem can then be converted to the 7-amino nucleus with methanolic hydrochloric acid.

The 7-amino-3-(bicyclicpyridinium-methyl)-3-cephem-4-carboxylate or the dihydrochloride salt thereof can also be obtained with cephalosporin C wherein the

amino and carboxy groups are protected. For example, cephalosporin C is first silylated with a conventional silylating reagent such as N-methyl-N-trimethylsilyltrifluoroacetamide to form the N-trimethylsilyl di-trimethylsilyl ester derivative. The latter is reacted with TMSI by the Bonjouklian method, and the 3-iodomethyl silylated derivative of cephalosporin C which is obtained is then allowed to react with the thienopyridine or the furopyridine and, following hydrolysis of the silyl groups, the compound of the formula (I) wherein R is α-aminoadipoyl is obtained. The α-amino-adipoyl side chain is cleaved by the N-deacylation procedure described above. In carrying out the N-deacylation, the amino group and the carboxy groups of the molecule are protected.

In carrying out the preparation of a 7-amino-3-(bicyclicpyridinium-methyl)-3-cephem-4-carboxylate with cephalosporin C, use can be made of the silylated 3-(bicyclicpyridinium-methyl) derivative obtained in the Bonjouklian method as described above. Since the amino group and the two carboxy groups are silylated, and thus protected, the N-deacylation can be carried out directly on this protected intermediate. During the final step of the N-deacylation, i.e.. following the formation of the imino ether of the side chain moiety, water is added to effect the hydrolysis of the silyl protecting group. The above-described preparation is illustrated by the following reaction scheme.

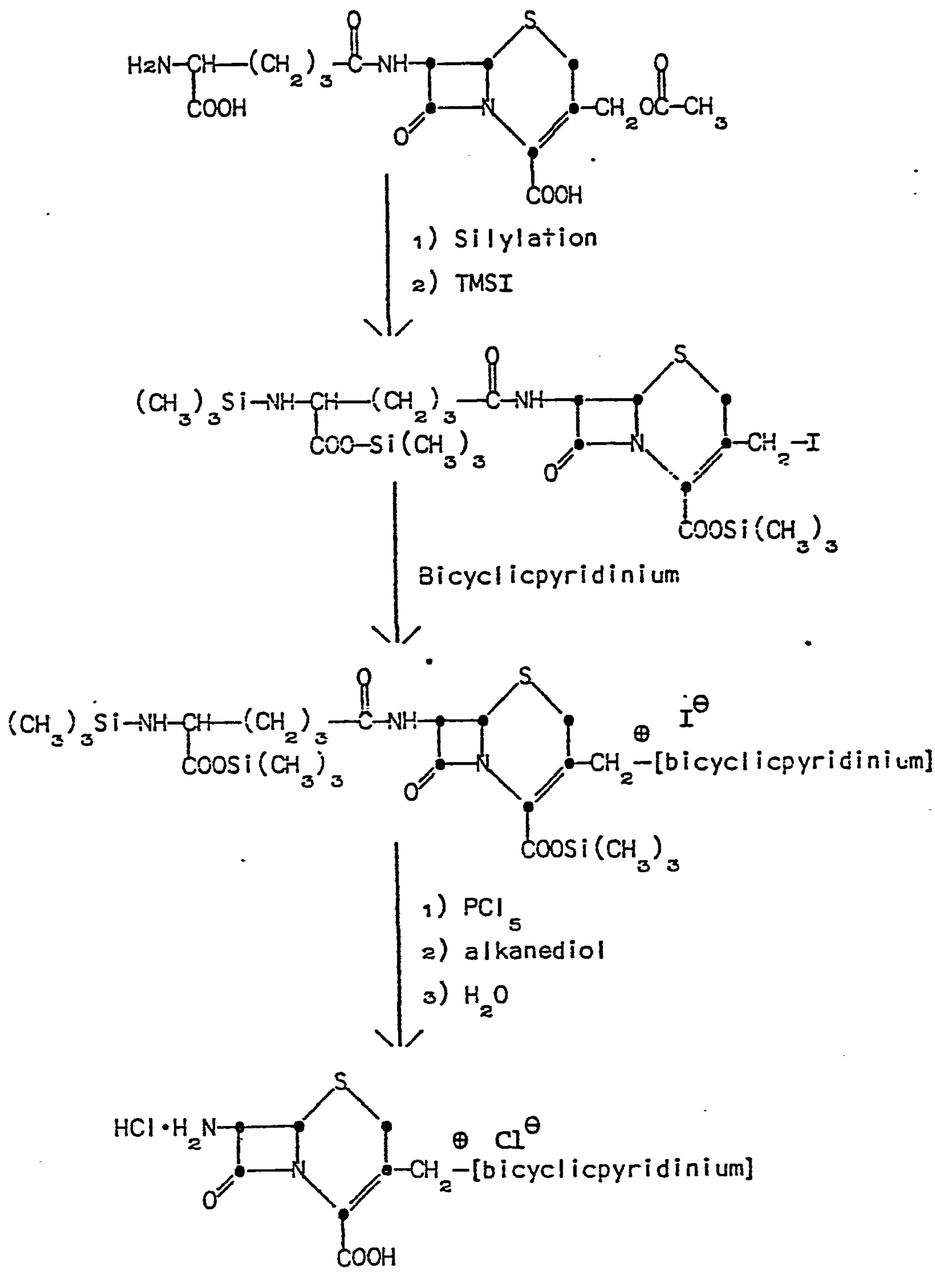
1) Silylation
2) TMSI
Bicyclicpyridinium
[bicyclicpyridinium]
1) PCl5
2) alkanediol
3) H2O
[bicyclicpyridinium]

Alternatively, the 7-amino-3-(bicyclicpyridinium-methyl) nucleus compound can be obtained with cephalosporin C having the amino group and the carboxy groups protected.

The 7-amino nucleus (formula (I), R = H) prepared by the N-deacylation method or via the N-formyl derivative can be acylated with an active carboxy derivative of a 2-(heterocyclic)-2-oximinoacetic acid represented by the formula

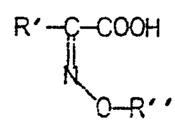

to provide an antibiotic compound of the formula (I). The N-acylation coupling reaction is carried out by acylation methods well-known in the art. Active derivatives of the carboxy group such as the so-called "active esters" can be used. Examples of active esters are those formed with the oximino acetic acid and hydroxybenzotriazole (HBT), or hydroxysuccinimide; and the esters formed with methyl chloroformate and isobutyl chloroformate. The acylation can also be carried out by employing the acid halide, e.g. the acid chloride, in the presence of an acid scavenger such as sodium bicarbonate or triethylamine.

The amino group of the amino-substituted heterocycles (R' in formula (I)) is desirably protected during the N-acylation of the 7-amino nucleus compound.

The compounds represented by the formula (II) wherein L is an acetoxy group can be prepared by known methods. For example, compounds wherein R' is the 2-aminothiazol-4-yl group are described by Heymes et al., U.S. Patent No. 4,152,432; compounds wherein R' is 2-aminopyridin-6-yl, 2-aminopyrimidin-5-yl, or 4-amino-pyrimidin-2-yl, are described in U.S. Patent No. 4,167,176; compounds wherein R' is 5-amino-1,2,4-thiadiazol-3-yl are described in European Patent Application No. 0,007,470; compounds wherein R'' is an N-substituted carbamoyl group can be prepared by the methods described in U.S. Patent No. 4,200,575; and compounds wherein R' is 3-aminopyrazol-5-yl, or pyra-zol-5-yl can be obtained as described in U.K. Patent Application No. 2,046,734.

Typically, the compounds of the formula (II) wherein L is acetoxy are prepared by the N-acylation of the 7-amino group of 7-aminocephalosporanic acid, or ester thereof, with the 2-(heterocyclic)-2-oximino-acetic acid by employing acylation methods known in the art. For example, the heterocyclic oximino-substituted acetic acid may be converted to an active ester such as the ester formed with hydroxybenzotriazole or hydroxysuccinimide, and the active ester used as the acylating moiety. Other active derivatives of the carboxylic acid such as the acid chloride, ester, or acid azide can be used in the acylation.

The compounds of the formula (II) wherein R' is a pyrazol-5-yl or 3-aminopyrazol-5-yl group are prepared by employing methods known in the art. The 2-(pyrazol-5-yl)-2-oximinoacetic acid or the 2-(3-aminopyrazol-5-yl)-2-oximinoacetic acid is prepared and converted to an active derivative of the carboxylic acid, for example, an active ester. The active ester

is coupled, via N-acylation, with 7-aminocephalosporanic acid and the 7-[2-(pyrazol-5-yl)-2-oximinoacetamido]-3-acetoxymethyl-3-cephem-4-carboxylic acid and 7-[2-(3-aminopyrazol-5-yl)-2-oximinoacetamido]-3-acetoxymethyl-3-cephem-4-carboxylic acid can be converted to the corresponding 3-iodomethyl silylated derivatives as described herein. The latter are reacted with the thieno- or furopyridine to provide a compound of the invention.

The pyrazole and aminopyrazole oximino substituted acetic acids can be prepared by employing synthetic methods known in the art. For example, the 2-(pyrazol-5-yl)-2-alkoxyiminoacetic acid may be prepared by heating in an inert hydrocarbon solvent the acetyl oximino compound of the formula A

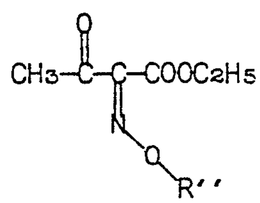

A

,

wherein R'' is other than hydrogen as defined above, with dimethylformamide dimethylacetal to form the dimethylaminomethylene oximino ester of the formula

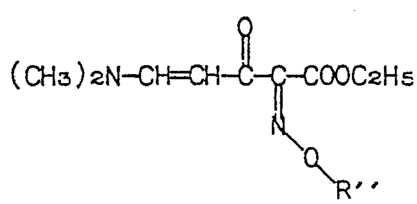

The latter can be reacted with hydrazine hydrate to provide the ethyl ester of 2-(pyrazol-5-yl)-2-alkoxy-

iminoacetic acid. The ester may be hydrolyzed to the free acid and the acid converted to an active ester for acylation.

The 2-(3-aminopyrazol-5-yl)-2-alkoxyimino-acetic acid can be prepared by reacting the compound of formula A with carbon disulfide and two equivalents of methyl iodide to form the intermediate compound of formula B

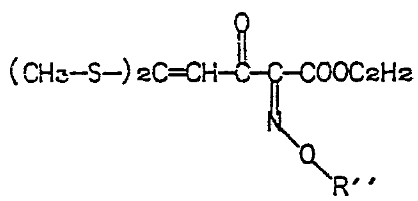

B

Intermediate B may be reacted with N-t-BOC hydrazine to provide compound C,

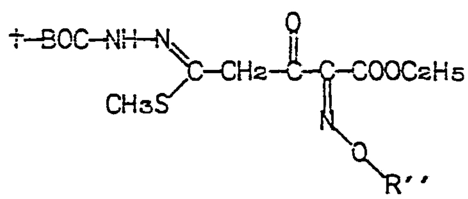

C

and C reacted with hydrazine hydrate to form 2-(3-t-BOC-hydrazinopyrazol-5-yl)-2-oximinoacetic acid ethyl ester D.

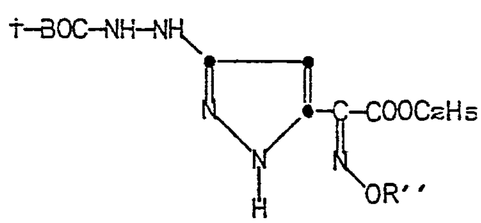

D

Compound D can be treated in the cold with trifluoroacetic acid to remove the t-BOC group and the 3-hydrazinopyrazole nitrosated with nitrous ($HNO_2$) acid in the cold to form 2-(3-azidopyrazol-5-yl)-2-oximinoacetic acid ethyl ester. The azido group can be reduced to an amino group by chemical reduction to provide the 2-(3-aminopyrazol-5-yl)-oximinoacetic acid ethyl ester. The ester can be hydrolyzed under alkaline conditions to the free acid.

The compounds of the invention have the same stereochemistry as known cephalosporin antibiotics. The 7-position side chain has the natural β-configuration (6R, 7R), while the oximino group in the side chain can exist in either the syn or anti forms or as a mixture of both. Compounds of the invention in either form are prepared by employing the 2-(heterocyclic)-2-oximinoacetic acid acylating moiety in the syn or anti form. Alternatively, mixtures of the syn and anti compounds of the formula (I) can be separated by chromatographic means such as by HPLC. The compounds in the syn form are preferred because of their greater activity.

The antibiotic compounds of the invention, and pharmaceutically-acceptable salts thereof, can be formulated into pharmaceutical compositions useful in the treatment of infectious diseases.

Accordingly, this invention provides pharmaceutical compositions comprising as the active ingredient a compound of formula (I), a pharmaceutically-acceptable salt thereof, associated with one or more pharmaceutically-acceptable excipients or carriers therefor.

Compositions for parenteral administration of the antibiotics or a salt thereof comprise the antibiotic or salt at a suitable concentration in a diluent such as Water-For-Injection, 5% dextrose, 0.9% saline, 5% glucose, or other pharmaceutically acceptable diluent. Such compositions are commonly prepared just prior to use as, for example, prior to intramuscular injection or intravenous infusion. An example of a composition of the invention useful for intramuscular administration comprises 0.5 g. of an antibiotic of the invention in 3 ml. of Water-For-Injection.

An example of a composition for intravenous use comprises between about 500 mg. to about 1 g. of an antibiotic of the invention in between about 50 ml. and 100 ml. of 0.9% saline. The intravenous solution can be prepared, for example, with a unit dosage formulation of the antibiotic in a plastic bag, by adding the diluent to the bag prior to infusion.

Pharmaceutical compositions of the invention also include unit dosage formulations. Such formulations comprise between about 200 mg. and about 10 g. of the antibiotic or a pharmaceutically acceptable nontoxic salt thereof in solid form in a sterile ampoule, vial or a plastic container such as a bag adapted for i.v. administration. The antibiotic may be amorphous or in the crystalline state. Such formulations may also contain a buffering agent, solubilizing agent, clarifying agent, stabilizing agent, or other excipient. An example of a pharmaceutical composition of

this invention for i.v. use comprises 500 mg. of the dry powder of the antibiotic or a pharmaceutically acceptable salt thereof in a 10 ml. sterile rubber-stoppered ampoule. Another such composition comprises 4 g. of dry powder of the antibiotic in a 100 ml. sterile ampoule. A further composition comprises 10 g. of the antibiotic as a dry powder in a sealed, sterile plastic pouch. Suppositories may also be used.

This invention also provides a method for treating a bacterial infection in a mammal (man or animal) which comprises administering in an effective dose of between about 100 mg. and about 2 g. of a compound of formula (I), or a pharmaceutically-acceptable salt thereof, to said mammal.

In practising the method of this invention, the antibiotic can be administered i.v., i.m. or s.c. The antibiotic may be administered in a single dose or in multiple doses, for example, one to four times daily. Administration of the dose by i.v. infusion can be carried out over an extended time interval, for example, with hospitalized patients over one to two hours. The method may also be practised by administering the dose simultaneously with an i.v. fluid such as plasma, a plasma extender, 5% dextrose, or glucose, by the piggy-back procedure. Commonly for i.v. infusion a unit dosage composition of the antibiotic in a plastic i.v. pouch is dissolved in the desired volume of diluent and the solution is infused.

A preferred treatment method of this invention comprises administering a preferred antibiotic of the invention as defined hereinabove.

The invention is further illustrated by the following examples wherein the abbreviations used have the following meanings.

TMSI is trimethylsilyliodide; THF is tetrahydrofuran; HPLC is high performance liquid chromatography; NMR is nuclear magnetic resonance spectrum; $DMSOd_6$ is deuterated dimethylsulfoxide; and the letters characterizing the NMR signals are as follows: s is singlet, d is doublet, q is quartet, m is multiplet, v is very, and b is broad.

The NMR spectra were run on a JEOL FX-90.

## Preparation 1

2-Carboxythieno[3,2-c]pyridine

Freshly distilled diisopropylamine (18.15 g., 180 mMole) was dissolved in sieve-dried tetrahydrofuran (200 ml.). The solution was cooled to -20°C., and stirred under a nitrogen blanket. *n* BuLi (176 mM) was then added, with care being taken to ensure that the temperature did not rise above -20°C. The temperature of the reaction mixture was then dropped to -70°C. using dry ice/acetone. A solution of thieno[3,2-c]-pyridine (150 mMole) in tetrahydrofuran was added dropwise taking care to ensure that the temperature did not rise above -65°C. The addition was complete after 20 minutes during which period stirring was continued. Carbon dioxide gas was then bubbled into the reaction mixture in such a way that the temperature remained below -60°C. for 30 minutes, -40°C. for 1 hour and, finally, under -15°C. for 30 minutes. Solvent was evaporated off *in vacuo*, and the residue was dissolved in water. The aqueous diisopropylammonium salt of the

title compound was washed (x3) with methylene chloride, and 120 ml. of 5N sodium hydroxide was then added. Cooling in ice water with stirring resulted in the precipitation of the sodium salt (24.8 g., after filtration under vacuum and drying).

This sodium salt was then dissolved in 10% aqueous methanol and the solution acidified to pH 6.0 with concentrated hydrochloric acid. After filtration and drying under vacuum there was obtained 19 g. of the title product.

NMR (DMSO$d_6$): signals at 8.1 (d, 1 proton), 8.2 (s, 1 proton), 8.5 (d, 1 proton), 9.1 (s, 1 (proton)

## Preparation 2

Furo[3,2-c]pyridine

To a partial solution of 117.3 g. (0.85 mole) of furan-2-acrylic acid in one-liter of sieve dried acetone was added with stirring under nitrogen triethylamine (101 g.) and the solution was cooled in an ice-alcohol bath. To the cold solution were added over about 20 minutes 119.35 g. (1.1 mole) of ethyl chloroformate. The rate of addition was such to maintain the temperature of the solution below 30°C. After the solution was stirred for about 15 minutes, a solution of 74.5 g. (1.3 mole) of sodium azide in 300 ml. of water was added at such a rate to maintain the temperature of the reaction mixture below about 10°C. The reaction mixture was stirred for one hour without external cooling and was poured onto 4 liters of crushed ice and the mixture stirred vigorously. The product was filtered, washed with ice water and vacuum dried to yield 131 g. of furan-2-acrylic acid azide.

A mixture of 150 ml. of diphenylmethane and 27 g. (145 mmole) of tributylamine was heated under nitrogen and reflux at 230°C. by means of a Wood's metal bath. To the hot mixture were added carefully and portionwise 25 g. of the azide prepared as described above. The temperature of the reaction mixture was held at about 225°C. to about 235°C. during the addition and thereafter at 245°C. for 30 minutes. The diphenylmethane was distilled from the reaction mixture under vacuum, the residue cooled and diluted with diethyl ether. The solid product was separated by filtration and recrystallized from hot water. The dried product, furopyridone of the formula

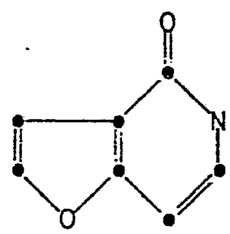

weighed 3.14 g. after drying.

The above 3.14 g. of the furopyridone was heated at the reflux temperature under nitrogen with stirring with 10 ml. of phosphorus oxychloride. Reflux was continued for 1.5 hours and the reaction mixture was then poured over ice. After the ice melted the product, 4-chlorofuro[3,2-c]pyridine, was extracted with diethyl ether. The extract was dried over sodium sulfate, filtered to remove drying agent, and evaporated to dryness to yield 3 g. of the product.

The 3 g. sample of the chlorofuropyridine was added to 35 ml. of glacial acetic acid and 7.5 g. of

zinc metal were added to the solution. The reaction mixture was refluxed under nitrogen for 4 hours. After the reaction was complete, the mixture was filtered and the filtrate containing the furo[3,2-c]pyridine product was evaporated to dryness to yield 5.1 g. of crude product. The product was purified by chromatography on a column packed with silica in methylene chloride. The column was eluted sequentially with 500 ml. of methylene chloride, 500 ml. of 2% acetone in methylene chloride, 500 ml. of 4% acetone in methylene chloride, one liter of 8% acetone in methylene chloride, 500 ml. of 12% acetone in methylene chloride, and finally with acetone. The fractions containing the product as shown by TLC were combined and evaporated. The semi-solid product was dissolved in 30 ml. of methylene chloride and 30 ml. of water were added. The pH of the mixture was adjusted to 8.4 with 1N sodium hydroxide and the organic layer separated. The organic layer was dried over sodium sulfate, filtered and evaporated to dryness to yield 1.43 g. of furo[3,2-c]pyridine.

## Preparation 3

Furo[2,3-c]pyridine

Furan-3-methanol (60 g.) was dissolved in 3 litres of sieve dried methylene chloride. To this vigorously power stirred solution under nitrogen at room temperature was added 240 g. of barium manganate. After stirring for 24 hours thin layer chromotography (3:1 petroleum ether/ether) showed starting material so another 60 g. of barium manganate was added, and stirring continued for a further 48 hours. Thin layer

chromotography showed loss of starting material so approximately half the methylene chloride was removed on a steam bath, and the crude reaction mixture filtered over celite. Distillation of this solution at 33 torr gave 47.3 g. of furan-3-aldehyde boiling at 64-67°C.

The Furan-3-aldehyde (9.6 g.) prepared above was dissolved in 200 ml. of chloroform. To this solution stirred at room temperature under nitrogen was added dropwise a solution of 36.56 g. (105 mm) of (carbethoxymethylene)triphenylphosphorane in 400 ml. of chloroform. After the addition was complete the reaction was heated to reflux for 1 hour, cooled to room temperature and evaporated under vacuum. Chromatography on silica gel using methylene chloride as the eluate gave 4.7 g. of furan-3-acrylic acid ethyl ester.

The ester prepared above was combined with like material prepared by the same route to give 6.8 g. of material. This material was dissolved in 60 ml. of absolute ethanol, magnetically stirred under nitrogen while 16.5 ml. of 5N aqueous sodium hydroxide was added dropwise. The instantly formed white precipitate was stirred at room temperature for 3 hours and evaporated to dryness under vacuum. The resulting solid was dissolved in de-ionized water, layered with methylene chloride and made acid to pH 1.0 with 6N HCl. The methylene chloride was separated and the aqueous acid washed twice more with methylene chloride, the organic extracts combined, dried with $Na_2SO_4$, and evaporated to give 5.3 g. of furan-3-acrylic acid.

To a solution of 10.2 g. of furan-3-acrylic acid produced by the above procedure in 100 ml. of sieve dried acetone, power stirred under nitrogen at ice/methanol temperature there was added 9.7 g. (97 mm.) of triethylamine in one portion. To the cold solution was added over a 20 minute period 9.33 g. (87 mm.) of iso-butylchloroformate in such a way that the temperature never exceeded 5°C.. After the solution had been stirred for 15 minutes a solution of 7.2 g. sodium azide in 30 ml. of water was added at such a rate as to maintain the temperature of the reaction mixture below 10°C. The reaction mixture was then stirred for 3 hours without external cooling and poured over 500 ml. of power stirred ice. The product was filtered, washed with ice water, and vacuum dried to give 9.1 g. of furan-3-acrylic acid azide.

A mixture of 36 g. of diphenylmethane and 9.72 g. of tri-butylamine was vigorously power stirred and heated to 235°C. under nitrogen with a woods metal bath. To the hot reaction mixture was added portionwise 9 g. of the acid azide, and the reaction mixture stirred at 235°C. for 30 minutes after the addition was distilled from the reaction mixture under vacuum, the residue cooled and diluted with diethyl ether. The solid product was separated by filtration and used without further purification in the next reaction. wt. 6.1 g.

4.56 g. of the furopyridone produced in the previous reaction was dissolved in 20 ml. of phosphorous oxychloride under nitrogen. After heating for 30 minutes in a 110°C. oil bath a solid began to come out of

solution. Thin layer chromatography of small portions of bicarbonate neutralized reaction mixture in 5% Acetone/$CH_2Cl_2$ showed no starting material and a new spot. The reaction mixture was cooled to ice temperature and poured into a vigorously stirred mixture of 300 ml. of Ice/100 ml. of ether. This mixture was made basic to pH 10 with 50% NaOH, the organics were separated and the aqueous base extracted 3X more with di-ethyl ether. Combination of the organic extracts followed by drying over $Na_2SO_4$ and evaporation gave solid 6-chlorofuro[23c]pyridine. 5.33 g. m.p. 64-66°C.

5 g. of the chlorofuro[23c] pyridine was dissolved in 50 ml. of power stirred glacial acetic acid under nitrogen. To this clear solution was added 11 g. of zinc dust and the reaction refluxed with a 115°C. oil bath for 1.5 hours. Thin layer chromatography in 5% acetone/methylene chloride showed no starting material and a new spot. The zinc dust was filtered off using celite and the acetic acid removed under vacuum. The resulting gum was dissolved in methylene chloride/-water and this mixture made basic to pH 10 with NaOH. After magnetically stirring for 30 minutes to a stable pH 10 the mixture was filtered over celite, the organic phase separated, dried over $Na_2SO_4$ and the solvent removed under vacuum. Distillation of this liquid in a short path distillation apparatus at 1 Torr. gave 2.15 g. of furo[2,3-c] pyridine.

Example 1

*syn*-7-[2-(2-Aminothiazol-4-yl)-2-methoxyiminoacetamido]-3-(thieno[2,3-b]pyridinium-7-ylmethyl)-3-cephem-4-carboxylate

To a suspension of 910 mg. of *syn*-7-[2-(2-aminothiazol-4-yl)-2-methoxyiminoacetamido]-3-acetoxymethyl-3-cephem-4-carboxylic acid in 4 ml. of chloroform were added 1.25 ml. of N-methyl-N-trimethylsilyltrifluoroacetamide, and the suspension was stirred for one hour when a solution of the silylated derivative was obtained. To the solution were added by pipette 800 µl. of TMSI and the reaction mixture was stirred for 15 minutes and was then evaporated. The residue of the silylated 3-iodomethyl derivative was dissolved in 4 ml. of acetonitrile and 175 µl. of THF were added to the solution by means of a syringe. The solution was stirred for 5 minutes after which a solution of 324 mg. of thieno[2,3-b]pyridine in 1 ml. of acetonitrile was added to the solution. The reaction mixture was stirred for 3 hours at room temperature and was then treated with 135 µl. of water. The product, 850 mg., was separated by filtration and was purified by reverse phase $C_{18}$ silica HPLC using acetonitrile-acetic acid-water, 5-2-93 percent by volume. There were obtained 125 mg. of the product as purified.

NMR($DMSOd_6$): signals at 9.6 (m, 2H), 9.05 (m, 1H), 8.31 (d, 1H), ca 8.2 (m, 1H), 7.89 (bs, 2H), 6.72 (s, 1H), ca 5.7 (bm, 3H), 5.08 (d, 1H), 3.79 (s, 3H), and ca 3.5 (m, 2H + water protons) delta.

Example 2

syn-7-[2-(2-Aminothiazol-4-yl)-2-methoxyiminoacetamido]-3-(thieno[3,2-b]pyridinium-4-ylmethyl)-3-cephem-4-carboxylate

The title compound was prepared by reacting syn-7-[2-(2-aminothiazol-4-yl)-2-methoxyiminoacetamido]-3-iodomethyl-3-cephem-4-carboxylic acid trimethylsilyl ester with thieno[3,2-b]pyridine by following the procedures described by Example 1.

NMR ($DMSOd_6$): signals at 3.2 (q, 2H, $C_2$-$H_2$), 3.8 (s, 3H, $OCH_3$), 5.0 (d, 1H, $C_6$-H), 5.6 (q, 1H, $C_7$-H), 5.8 (q, 2H, $C_{3'}$-H), 6.65 (s, 1H, thiazole H), 7.2 (s, 2H, $NH_2$), 7.2-9.6 (multi signals for thienopyridine), and 9.6 (d, 1H, NH) delta.

Example 3

syn-7-[2-(2-Aminothiazol-4-yl)-2-methoxyiminoacetamido]-3-(thieno[2,3-c]pyridinium-6-ylmethyl-3-cephem-4-carboxylate was prepared with trimethylsilylated syn-7-[2-(2-aminothiazol-4-yl)-2-methoxyiminoacetamido]-3-iodomethyl-3-cephem-4-carboxylic acid and thieno[2,3-c]pyridine by the procedure of Example 1.

NMR ($DMSOd_6$): signals at ca 3.2 (q, 2H, $C_2$-$H_2$, masked by HOD), 3.8 (s, 3H, $OCH_3$), 5.05 (d, 1H, $C_6$-H), 5.2 (s), 5.6 (q, 1H, $C_7$-H), 5.9 (s), 6.7 (s, 1H, thiazole-H), 7.2 (s, 2H, $NH_2$), and 7.95, d; 8.55, d; 9.45, m; 10.45, s (thienopyridinium H) delta.

Example 4

syn-7-[2-(2-Aminothiazol-4-yl)-2-methoxyiminoacetamido]-3-(thieno[3,2-c]pyridinium-5-ylmethyl)-3-cephem-4-carboxylate was prepared by reacting trimethylsilylated syn-7-[2-(2-aminothiazol-4-yl)-2-methoxyiminoacet-

amido]-3-iodomethyl-3-cephem-4-carboxylic acid with thieno[3,2-c]pyridine by following the procedures described by Example 1.

NMR ($DMSOd_6$): signals at 3.3 (q, 2H, $C_2$-H), 3.8 (s, 3H, $OCH_3$), 5.1 (d, 1H, $C_6$-H), 5.7 (m, 2H, $C_{3'}$-H), 6.7 (s, 1H, thiazole-H), 7.2 (s, 2H, $NH_2$), and 8.0-10.0 (thienopyridinium H) delta.

UV: λmax. 255 n.m.; ε: 19,557.

Using procedures similar to those described above in Example 1 the following further compounds were prepared.

## Example 5

syn-7-[2-(2-Aminothiazol-4-yl)-2-(2-carboxyprop-2-yl)-oxyiminoacetamido]-3-(thieno[3,2-c]pyridinium-5-yl-methyl)-3-cephem-4-carboxylate

NMR ($DMSOd_6$): signals at 9.98 (s, 1H), 9.45 (d, 1H), 9.25 (d, 1H), 8.8 (d, 1H), 8.35 (d, 1H), 7.95 (d, 1H), 7.25 (s, 1H), 6.7 (s, 1H), 5.7 (q, 1H), 5.5 (q, 2H), 5.1 (d, 1H), 3.4 (q, 2H), 1.4 (s, 6H) delta.

UV: λmax. 238 n.m.; ε: 25,726.

## Example 6

syn-7-[2-(2-Aminothiazol-4-yl)-2-methoxyiminoacetamido]-3-(2-methylthieno[3,2-c]pyridinium-5-ylmethyl)-3-cephem-4-carboxylate

NMR ($DMSOd_6$): signals at 9.8 (s, 1H), 9.5 (d, 1H), 9.2 (d, 1H), 8.65 (d, 1H), 7.6 (s, 1H), 7.15 (s, 2H), 6.7 (s, 1H), 5.45 (m, 3H), 5.05 (d, 1H), 3.8 (s, 3H), 3.3 (q, 2H), 2.7 (s, 3H) delta.

UV: λmax. 242 n.m.; ε: 31,614.

Example 7

syn-7-[2-(2-Aminothiazol-4-yl)-2-methoxyiminoacetamido]-3-(2-carboxythieno[3,2-c]pyridinium-5-ylmethyl)-3-cephem-4-carboxylate

NMR ($DMSOd_6$): signals at 9.7 (s, 1H), 9.5 (d, 1H), 9.0 (d, 1H), 8.7 (d, 1H), 8.1 (s, 1H), 7.1 (s, 2H), 6.7 (s, 1H), 5.7 (q, 1H), 5.3 (d, 2H), 5.1 (d, 1H), 3.8 (s, 3H), 3.4 (q, 2H) delta.

UV: λmax. 245 n.m.; ε: 46,000

Example 8

syn-7-[2-(2-Aminothiazol-4-yl)-2-methoxyiminoacetamido]-3-(3-bromothieno[3,2-c]pyridinium-5-ylmethyl)-3-cephem-4-carboxylate

NMR ($DMSOd_6$): signals at 10.04 (s, 1H), 9.45 (m, 2H), 8.8 (d, 1H), 8.5 (s, 1H), 7.15 (s, 2H), 6.65 (s, 1H), 5.6 (q, 1H), 5.5 (q, 2H), 5.05 (d, 1H), 3.75 (s, 3H), 3.3 (q, 2H) delta

UV: λmax. 244 n.m.; ε: 33,500.

Example 9

syn-7-[2-(Aminothiazol-4-yl)-2-methoxyiminoacetamido]-3-(2-methoxycarbonylthieno[3,2-c]pyridinium-5-ylmethyl)-3-cephem-4-carboxylate

NMR ($DMSOd_6$): signals at 10.1 (s, 1H), 9.45 (m, 2H), 8.8 (d, 1H), 8.55 (s, 1H), 7.15 (s, 2H), 6.65 (s, 1H), 5.6 (q, 1H), 5.45 (q, 2H), 5.05 (d, 1H), 3.95 (s, 3H), 3.8 (s, 3H), 3.3 (q, 2H) delta

UV: λmax. 243 n.m.; ε: 52,500

Example 10

syn-7-[2-(2-Aminothiazol-4-yl)-2-methoxyiminoacetamido]-3-(furo[3,2-c]pyridinium-5-ylmethyl)-3-cephem-4-carboxylate

A suspension of 910 mg. (2 mmole) of _syn_-7-[2-(2-aminothiazol-4-yl)-2-methoxyiminoacetamido]-3-acetoxymethyl-3-cephem-4-carboxylic acid in 5 ml. of methylene chloride was treated with 1.24 ml. (7 mmole) of N-methyl-N-trimethylsilyltrifluoroacetamide (MSTFA) and the mixture warmed to about 40°C. to achieve silylation. After the solution was formed, the solution was cooled to room temperature and 0.77 ml. (5.4 mmole) of TMSi was added by syringe. The reaction mixture was stirred at room temperature under nitrogen for 3/4 hour and was then evaporated to a brown oil. The oil was dissolved in 5 ml. of acetonitrile and 0.73 ml. (9 mmole) of THF were added. The solution was stirred for 10 minutes. To this solution was added a solution of 286 mg. (2.4 mmole) of furo[3,2-c]pyridine in 5 ml. of acetonitrile to which had been added 0.43 ml. (2.4 mmole) MSTFA. The combined solutions were stirred at room temperature under nitrogen for 2 hours. The reaction mixture was diluted with diethyl ether and 3 drops of water were added to precipitate the product as a thick, tan solid. The mixture was sonnicated, filtered, washed with diethyl ether, and dried at 40°C. for 1 hour under vacuum to yield 1.28 g. of the crude cephalosporin product. The product was purified via preparative HPLC using 5% acetonitrile, 2% acetic acid, and 93% water. There were obtained 14 mg. of the 2-cephem product and 580 mg. of the 3-cephem product as a white powder.

Example 11

_syn_-7-[2-(2-Aminothiazol-4-yl)-2-methoxyiminoacetamido-3-(2-methylfuro[3,2-b]pyridinium-4-ylmethyl)-3-cephem-4-carboxylate

syn-7-[2-(2-Aminothiazol-4-yl)-2-methoxyiminoacetamido]-3-acetoxymethyl-3-cephem-4-carboxylic acid, 910 mg. (2 mmole) were suspended in 5 ml. of methylene chloride and the suspension treated with 1.24 ml. (7 mmole) of MSTFA under nitrogen. The suspension was heated at 40°C. until a solution of the silylated derivative was formed (5 minutes). The solution was cooled to room temperature and 0.77 ml. (5.4 mmole) of TMSI was added with a syringe. The solution was stirred for about 45 minutes at room temperature under nitrogen to form the silylated 3-iodomethyl derivative. Thereafter a solution of 319 mg. (2.4 mmole) of 2-methylfuro[3,2-b]pyridine in 10 ml. of acetonitrile was added to the reaction solution and the mixture was stirred for about 3 hours. The reaction mixture was then diluted with diethyl ether and two drops of water were added. The thick tan precipitate which formed was sonnicated, filtered, washed with diethyl ether and dried under vacuum at 40°C. to give 1.03 g. of the crude product. The product was purified over $C_{18}$ silica gel reverse phase chromatography using 5% acetonitrile:2% acetic acid:93% water, v:v:v to give 348 mg. of the purified product.

NMR (90 MHz, $DMSOd_6$) 9.45 (d, 1H), 9.3 (d, 1H), 8.7 (d, 1H), 7.9 (m, 1H), 7.75 (s, 1H), 7.15 (s, 2H), 6.65 (s, 1H), 5.6 (m, 3H), 5.0 (d, 1H), 3.8 (s, 3H), 3.4 (q, 2H), 2.7 (s, 3H) delta.

UV: λmax 256 nm; ε: 17,924.

Example 12

syn-7-[2-(2-Aminothiazol-4-yl)-2-methoxyiminoacetamido]-3-(2-methylfuro[2,3-c]pyridinium-6-ylmethyl)-3-cephem-4-carboxylate

By following the procedures and conditions of the preceding Example 11, 910 mg. (2 mmole) of the same starting material was converted to the silylated 3-iodomethyl derivative and the latter reacted with 2-methylfuro[2,3-c]pyridine. The product was precipitated, recovered and purified by procedures described in Example 18 to give 526 mg. of the purified title compound.

NMR (90 MHz, DMSO-$d_6$) 10.03 (s, 1H), 9.45 (d, 1H), 9.15 (d, 1H), 8.15 (d, 1H), 7.1 (s, 3H), 6.65 (s, 1H), 5.6 (m, 2H), 5.05 (m, 2H), 3.75 (s, 3H), 3.3 (q, 2H), 2.65 (s, 3H) delta.

UV: λmax. 268 n.m.; ε: 22,278.

Similarly prepared were:

Example 13

syn-7-[2-(2-Aminothiazol-4-yl)-2-methoxyiminoacetamido]-3-(furo[2,3-b]pyridinium-7-ylmethyl)-3-cephem-4-carboxylate

NMR (DMSO$d_6$): signals at 9.4 (m, 2H), 8.1 (m, 2H), 7.2 (s, 2H), 6.7 (s, 1H), 5.65 (m, 3H), 5.05 (d, 1H), 3.8 (s, 3H), 3.2 (s, 2H) delta.

UV: λmax. 232 n.m.; ε: 19,100

λmax. 252 n.m.; ε: 18,000

Example 14

syn-7-[2-(2-Aminothiazol-4-yl)-2-methoxyiminoacetamido]-3-(furo[3,2-b]pyridinium-4-ylmethyl)-3-cephem-4-carboxylate

NMR ($DMSOd_6$): signals at 9.4 (m, 2H), 8.85 (m, 2H), 8.0 (m, 2H), 7.15 (s, 2H), 6.65 (s, 1H), 5.6 (m, 3H), 4.95 (d, 1H), 3.7 (s, 3H), 3.15 (q, 2H) delta.

UV: λmax. 260 n.m.; ε: 22,844

Example 15

syn-7-[2-(2-Aminothiazol-4-yl)-2-methoxyiminoacetamido]-3-(furo[2,3-c]pyridinium-6-ylmethyl)-3-cephem-4-carboxylate

NMR ($DMSOd_6$): signals at 10.2 (s, 1H), 9.5 (d, 1H), 9.3 (d, 1H), 8.8 (s, 1H), 8.35 (d, 1H), 7.45 (s, 1H), 7.15 (s, 2H), 6.65 (s, 1H), 5.65 (q, 1H), 5.4 (q, 2H), 5.05 (d, 1H), 3.75 (s, 3H), 3.3 (q, 2H) delta.

UV: λmax. 263 n.m.; ε: 20,642.

Example 16

syn-7-[2-(2-Aminothiazol-4-yl)-2-methoxyiminoacetamido]-3-(thieno[3,2-c]pyridinium-5-ylmethyl)-3-cephem-4-carboxylate

7-Aminocephalosporanic acid (2.7 g, 10 mMole) and thieno[3,2-c]pyridine (3.0 g, 10 mMole) in the form of its tosylate salt were suspended in a mixture of water (25 ml) and acetonitrile (25 ml). The pH was adjusted to 7.3 with NaOH. The reaction mixture was then heated in an oil bath at 70°C. for 2 3/4 hours and then cooled to 0°C. at which time the pH was 6.8. The pH of the reaction mixture, was adjusted to 7.5 with further 2N NaOH. The product of the above procedure was:

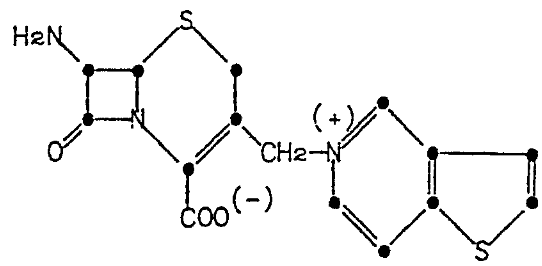

However, this product was not isolated but was used in situ in the next step in the reaction. In other words, the 7-amino nucleus depicted above was immediately acylated by adding the active ester syn-1-[(2-amino-4-thiazolyl)(methoxyimino)acetyl]-3-hydroxy-1H-benzotriazolium, hydroxide, inner salt (3.0 g.) in solid form to the reaction mixture. After 1/2 hour, the pH was adjusted to 7.3 with 2N NaOH and the reaction mixture allowed to stand for 16 hours. After standard work-up there was obtained 900 mg. of the title product.

NMR ($DMSOd_6$): signals at 3.3 (q, 2H), 3.8 (s, 3H), 5.1 (d, 1H), 5.7 (m, 2H), 6.7 (s, 1H), 7.2 (s, 2H), and 8-10 (thienopyridinium H) delta

UV: λmax. 255 n.m.; ε: 19,557.

Similarly prepared were:

Example 17

syn-7-[2-(2-Aminothiazol-4-yl)-2-methoxyiminoacetamido]-3-(thieno[2,3-b]pyridinium-7-ylmethyl)-3-cephem-4-carboxylate

NMR ($DMSOd_6$): signals at 9.6 (m, 2H), 9.05 (m, 1H), 8.31 (d, 1H), ca 8.2 (m, 1H), 7.89 (bs, 2H), 6.72 (s, 1H), ca 5.7 (bm, 3H), 5.08 (d, 1H), 3.79 (s, 3H), and ca 3.5 (m, 2H + water protons) delta.

### Example 18

syn-7-[2-(2-Aminothiazol-4-yl)-2-methoxyiminoacetamido]-3-(thieno[3,2-b]pyridinium-4-ylmethyl)-3-cephem-4-carboxylate

NMR ($DMSOd_6$): signals at 3.2 (q, 2H, $C_2$-$H_2$), 3.8 (s, 3H, $OCH_3$), 5.0 (d, 1H, $C_6$-H), 5.6 (q, 1H, $C_7$-H), 5.8 (q, 2H, $C_{3'}$-H), 6.65 (s, 1H, thiazole H), 7.2 (s, 2H, $NH_2$), 7.2-9.6 (multi signals for thienopyridine), and 9.6 (d, 1H, NH) delta.

### Example 19

syn-7-[2-(2-Aminothiazol-4-yl)-2-methoxyiminoacetamido]-3-(thieno[2,3-c]pyridinium-6-ylmethyl-3-cephem-4-carboxylate

NMR ($DMSOd_6$): signals at ca 3.2 (q, 2H, $C_2$-$H_2$, masked by HOD), 3.8 (s, 3H, $OCH_3$), 5.05 (d, 1H, $C_6$-H), 5.2 (s), 5.6 (q, 1H, $C_7$-H), 5.9 (s), 6.7 (s, 1H, thiazole-H), 7.2 (s, 2H, $NH_2$), and 7.95, d; 8.55, d; 9.45, m; 10.45, s (thienopyridinium H) delta.

### Example 20

syn-7-[2-(2-Aminothiazol-4-yl)-2-methoxyiminoacetamido]-3-(thieno[3,2-c]pyridinium-5-ylmethyl)-3-cephem-4-carboxylate

NMR ($DMSOd_6$): signals at 3.3 (q, 2H, $C_2$-H), 3.8 (s, 3H, $OCH_3$), 5.1 (d, 1H, $C_6$-H), 5.7 (m, 2H, $C_{3'}$-H), 6.7 (s, 1H, thiazole-H), 7.2 (s, 2H, $NH_2$), and 8.0-10.0 (thienopyridinium H) delta.

UV: λmax. 255 n.m.; ε: 19,557.

Example 22

syn-7-[2-(2-Aminothiazol-4-yl)-2-(2-carboxyprop-2-yl)-oxyiminoacetamido]-3-(thieno[3,2-c]pyridinium-5-yl-methyl)-3-cephem-4-carboxylate

NMR ($DMSOd_6$): signals at 9.98 (s, 1H), 9.45 (d, 1H), 9.25 (d, 1H), 8.8 (d, 1H), 8.35 (d, 1H), 7.95 (d, 1H), 7.25 (s, 1H), 6.7 (s, 1H), 5.7 (q, 1H), 5.5 (q, 2H), 5.1 (d, 1H), 3.4 (q, 2H), 1.4 (s, 6H) delta.

UV: λmax. 238 n.m.; ε: 25,726.

Example 23

syn-7-[2-(2-Aminothiazol-4-yl)-2-methoxyiminoacetamido]-3-(2-methylthieno[3,2-c]pyridinium-5-ylmethyl)-3-cephem-4-carboxylate

NMR ($DMSOd_6$): signals at 9.8 (s, 1H), 9.5 (d, 1H), 9.2 (d, 1H), 8.65 (d, 1H), 7.6 (s, 1H), 7.15 (s, 2H), 6.7 (s, 1H), 5.45 (m, 3H), 5.05 (d, 1H), 3.8 (s, 3H), 3.3 (q, 2H), 2.7 (s, 3H) delta.

UV: λmax. 242 n.m.; ε: 31,614.

Example 24

syn-7-[2-(2-Aminothiazol-4-yl)-2-methoxyiminoacetamido]-3-(2-carboxythieno[3,2-c]pyridinium-5-ylmethyl)-3-cephem-4-carboxylate

NMR ($DMSOd_6$): signals at 9.7 (s, 1H), 9.5 (d, 1H), 9.0 (d, 1H), 8.7 (d, 1H), 8.1 (s, 1H), 7.1 (s, 2H), 6.7 (s, 1H), 5.7 (q, 1H), 5.3 (d, 2H), 5.1 (d, 1H), 3.8 (s, 3H), 3.4 (q, 2H) delta

UV: λmax. 245 n.m.; ε: 46,000

Example 25

syn-7-[2-(2-Aminothiazol-4-yl)-2-methoxyiminoacetamido]-3-(3-bromothieno[3,2-c]pyridinium-5-ylmethyl)-3-cephem-4-carboxylate

NMR ($DMSOd_6$): signals at 10.04 (s, 1H), 9.45 (m, 2H), 8.8 (d, 1H), 8.5 (s, 1H), 7.15 (s, 2H), 6.65 (s, 1H), 5.6 (q, 1H), 5.5 (q, 2H), 5.05 (d, 1H), 3.75 (s, 3H), 3.3 (q, 2H) delta

UV: λmax. 244 n.m.; ε: 33,500.

Example 26

syn-7-[2-(Aminothiazol-4-yl)-2-methoxyiminoacetamido]-3-(2-methoxycarbonylthieno[3,2-c]pyridinium-5-ylmethyl)-3-cephem-4-carboxylate

NMR ($DMSOd_6$): signals at 10.1 (s, 1H), 9.45 (m, 2H), 8.8 (d, 1H), 8.55 (s, 1H), 7.15 (s, 2H), 6.65 (s, 1H), 5.6 (q, 1H), 5.45 (q, 2H), 5.05 (d, 1H), 3.95 (s, 3H), 3.8 (s, 3H), 3.3 (q, 2H) delta

UV: λmax. 243 n.m.; ε: 52,500

Example 27

syn-7-[2-(2-Aminothiazol-4-yl)-2-methoxyiminoacetamido]-3-(furo[3,2-c]pyridinium-5-ylmethyl)-3-cephem-4-carboxylate

Example 28

syn-7-[2-(2-Aminothiazol-4-yl)-2-methoxyiminoacetamido-3-(2-methylfuro[3,2-b]pyridinium-4-ylmethyl)-3-cephem-4-carboxylate

NMR (90 MHz, $DMSOd_6$) 9.45 (d, 1H), 9.3 (d, 1H), 8.7 (d, 1H), 7.9 (m, 1H), 7.75 (s, 1H), 7.15 (s, 2H), 6.65 (s,

2H), 6.65 (s, 1H), 5.6 (m, 3H), 5.0 (d, 1H), 3.8 (s, 3H), 3.4 (q, 2H), 2.7 (s, 3H) delta.

UV: λmax 256 nm; ε: 17,924.

Example 29

syn-7-[2-(2-Aminothiazol-4-yl)-2-methoxyiminoacetamido]-3-(2-methylfuro[2,3-c]pyridinium-6-ylmethyl)-3-cephem-4-carboxylate

NMR (90 MHz, DMSO-$d_6$) 10.03 (s, 1H), 9.45 (d, 1H), 9.15 (d, 1H), 8.15 (d, 1H), 7.1 (s, 3H), 6.65 (s, 1H), 5.6 (m, 2H), 5.05 (m, 2H), 3.75 (s, 3H), 3.3 (q, 2H), 2.65 (s, 3H) delta.

UV: λmax. 268 n.m.; ε: 22,278.

Example 30

syn-7-[2-(2-Aminothiazol-4-yl)-2-methoxyiminoacetamido]-3-(furo[2,3-b]pyridinium-7-ylmethyl)-3-cephem-4-carboxylate

NMR (DMSO$d_6$): signals at 9.4 (m, 2H), 8.1 (m, 2H), 7.2 (s, 2H), 6.7 (s, 1H), 5.65 (m, 3H), 5.05 (d, 1H), 3.8 (s, 3H), 3.2 (s, 2H) delta.

UV: λmax. 232 n.m.; ε: 19,100
λmax. 252 n.m.; ε: 18,000

Example 31

syn-7-[2-(2-Aminothiazol-4-yl)-2-methoxyiminoacetamido]-3-(furo[3,2-b]pyridinium-4-ylmethyl)-3-cephem-4-carboxylate

NMR (DMSO$d_6$): signals at 9.4 (m, 2H), 8.85 (m, 2H), 8.0 (m, 2H), 7.15 (s, 2H), 6.65 (s, 1H), 5.6

(m, 3H), 4.95 (d, 1H), 3.7 (s, 3H), 3.15 (q, 2H) delta.

UV: λmax. 260 n.m.; ε: 22,844

Example 32

<u>syn</u>-7-[2-(2-Aminothiazol-4-yl)-2-methoxyiminoacetamido]-3-(furo[2,3-c]pyridinium-6-ylmethyl)-3-cephem-4-carboxylate

NMR ($DMSOd_6$): signals at 10.2 (s, 1H), 9.5 (d, 1H), 9.3 (d, 1H), 8.8 (s, 1H), 8.35 (d, 1H), 7.45 (s, 1H), 7.15 (s, 2H), 6.65 (s, 1H), 5.65 (q, 1H), 5.4 (q, 2H), 5.05 (d, 1H), 3.75 (s, 3H), 3.3 (q, 2H) delta.

UV: λmax. 263 n.m.; ε: 20,642.

CLAIMS

1. A cephalosporin derivative of formula (I):

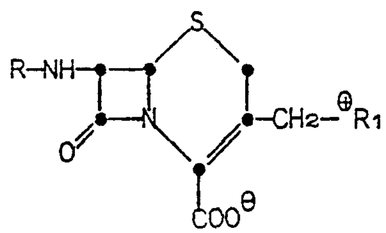

(I)

wherein R is hydrogen or an acyl group represented by the formula:

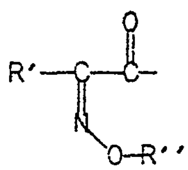

wherein R' is a 5- or 6-membered heterocyclic ring represented by the formulae:

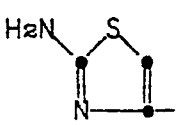 , 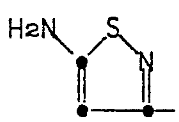 , 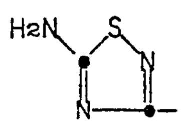 , 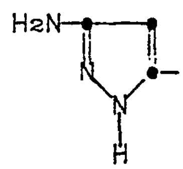 ,

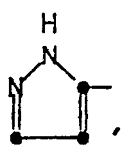 , 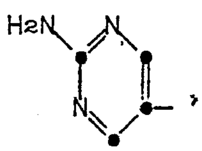 , 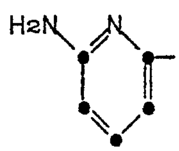 , or 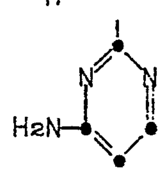 ;

R'' is hydrogen, $C_1$-$C_4$ alkyl, a carboxy-substituted alkyl or carboxy-substituted cycloalkyl group represented by the formula

$$-\overset{a}{\underset{b}{C}}-(CH_2)_n-COR'''$$

wherein n is 0-3, a and b when taken separately are independently hydrogen or $C_1$-$C_3$ alkyl, or when taken together with the carbon to which they are attached form a $C_3$-$C_7$ carbocyclic ring; R''' is hydroxy, amino, $C_1$-$C_4$ alkoxy, or OR° wherein R° is a carboxy-protecting ester group;

or R'' is a carbamoyl group represented by the formula

$$-\overset{O}{\overset{\|}{C}}-NHR''''$$

wherein R'''' is $C_1$-$C_4$ alkyl, phenyl, or $C_1$-$C_3$ alkyl substituted by phenyl; $^{\oplus}R_1$ is a bicyclic-pyridinium group selected from the group consisting of a thienopyridinium group represented by the formulae

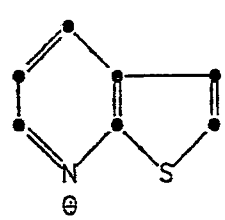 , 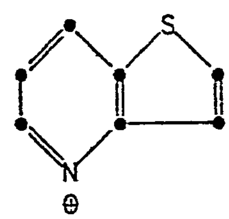

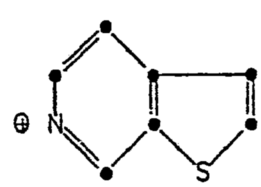 , 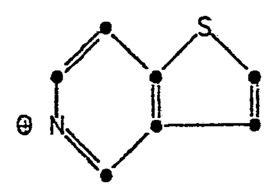

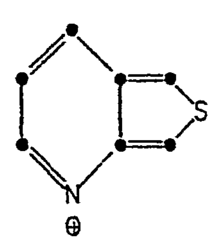 , 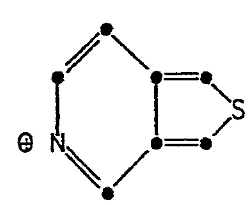

or a furopyridinium group represented by the formulae

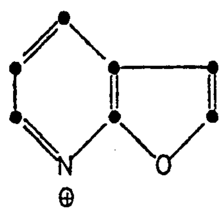 , 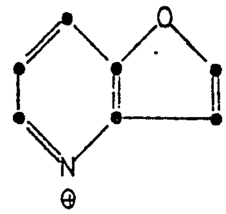 ,

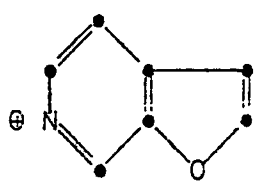 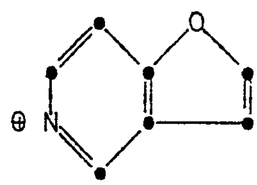 ,

where in the above formulae either or both of the hetero rings is optionally substituted by one or two substituents independently selected from $C_1$-$C_4$ alkyl, halogen, trifluoromethyl, carboxy, carbamoyl, $-SO_3H$, hydroxy, $C_1$-$C_4$ alkoxy, amino, mono $C_1$-$C_4$ alkylamino, di($C_1$-$C_4$) alkylamino, $C_{2-4}$ alkanoylamino, aminosulphonyl, cyano, $C_1$-$C_4$ alkoxycarbonyl or a group of formula

$$-\overset{O}{\overset{\parallel}{C}}-NR_5-OR_6$$

where $R_5$ and $R_6$ are independently hydrogen or $C_1$-$C_4$ alkyl; or a pharmaceutically-acceptable salt thereof.

2. A cephalosporin derivative of formula (I), or a pharmaceutically-acceptable salt thereof, as claimed in claim 1 in which R is

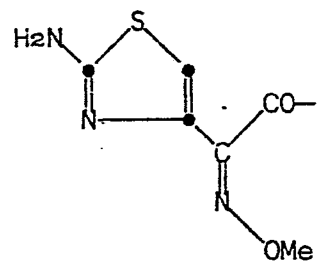

in which the oxime has a syn-configuration.

3. A cephalosporin derivative of formula (I), or a pharmaceutically-acceptable salt thereof, as claimed in claim 1 or 2, wherein either or both of the thieno- or furopyridinium rings is optionally substituted by one or two $C_1$-$C_4$ alkyl, fluoro, chloro, bromo, carboxy, carbamoyl or $C_1$-$C_4$ alkoxycarbonyl groups.

4. A cephalosporin derivative of formula (I), or a pharmaceutically-acceptable salt thereof, as claimed in any one of claims 1 to 3, wherein either or both of the thieno or furopyridinium rings is substituted by carboxy.

5. syn-7-[2-(2-Aminothiazol-4-yl)-2-methoxyiminoacetamido]-3-(2-carboxythieno[3,2-c]pyridinium-5-ylmethyl)-3-cephem-4-carboxylate.

6. A cephalosporin derivative of formula (I), or a pharmaceutically-acceptable salt thereof, as claimed in claim 1 or 2, wherein $^{(+)}R_1$ is an unsubstituted thienopyridinium group.

7. A process for preparing a cephalosporin derivative of formula (I), or a pharmaceutically-acceptable salt thereof, as claimed in any one of claims 1 to 6, which comprises:

(a) condensing a compound of formula:

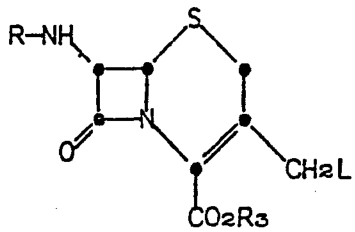 (II)

where L is a leaving group and $R_3$ is hydrogen or an ester protecting group; with a thieno or furopyridine of formula $R_1$, followed, in the case where $R_3$ is an ester protecting group, by removal of that group and any protecting groups which may be present in the R-substituent;

(b) acylating a compound of formula

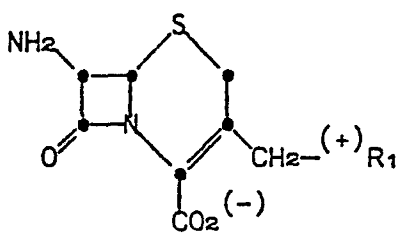 (III)

, where $R_1$ is as defined above, or a salt or 4'-ester ($R_3$) thereof, with an active carboxy derivative of an acid of formula

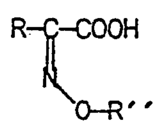

followed, in the case where the compound of formula (III) is in the form of an ester, by removal of the $R_3$ group, together with any amino protecting groups which may be present in R-substituent.

8. A cephalosporin derivative of formula (I) in which R is an acyl group, or a pharmaceutically-acceptable salt thereof, as claimed in any one of claims 1 to 6, for use as an antibiotic.

9. A pharmaceutical formulation which comprises as an active ingredient a compound of formula (I) in which R is an acyl group, or a pharmaceutically-acceptable salt thereof, as claimed in any one of claims 1 to 6, associated with one or more pharmaceutically-acceptable excipients or carriers therefor.

10. 2-Carboxy-thieno (3,2-c)pyridine"

## CLAIMS

1. A process for preparing a cephalosporin derivative of formula (I):

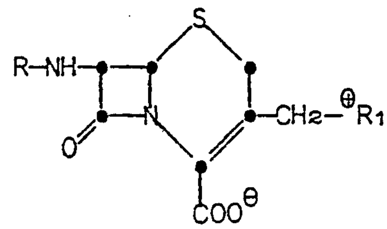 (I)

wherein R is hydrogen or an acyl group represented by the formula:

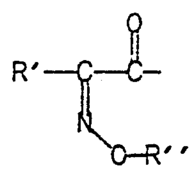

wherein R' is a 5- or 6-membered heterocyclic ring represented by the formulae:

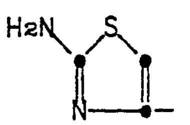 , 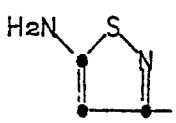 ; 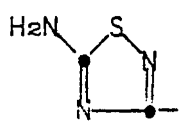 , 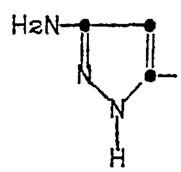 ,

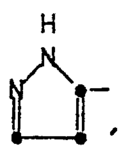 , 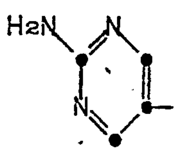 , 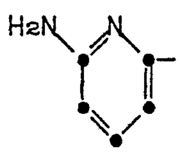 , or 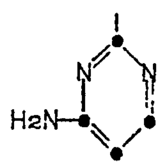 ;

R'' is hydrogen, $C_1$-$C_4$ alkyl, a carboxy-substituted alkyl or carboxy-substituted cycloalkyl group represented by the formula

$$-\overset{a}{\underset{b}{C}}-(CH_2)_n-COR'''$$

wherein n is 0-3, a and b when taken separately are independently hydrogen or $C_1$-$C_3$ alkyl, or when taken together with the carbon to which they are attached form a $C_3$-$C_7$ carbocyclic ring; R''' is hydroxy, amino, $C_1$-$C_4$ alkoxy, or OR° wherein R° is a carboxy-protecting ester group;

or R'' is a carbamoyl group represented by the formula

$$-\overset{\overset{\displaystyle O}{\|}}{C}-NHR''''$$

wherein R'''' is $C_1$-$C_4$ alkyl, phenyl, or $C_1$-$C_3$ alkyl substituted by phenyl; ${}^{\oplus}R_1$ is a bicyclic-pyridinium group selected from the group consisting of a thienopyridinium group represented by the formulae

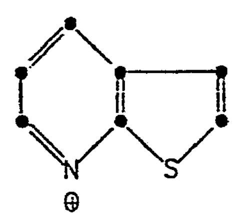

,

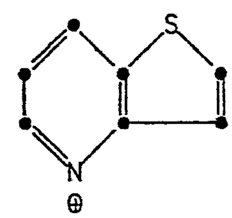

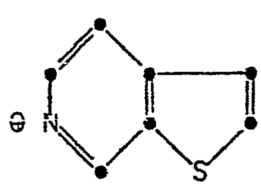

,

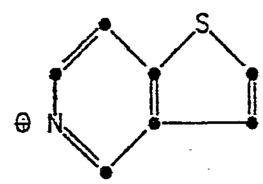

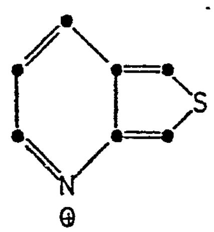

,

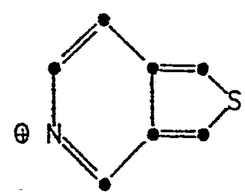

or a furopyridinium group represented by the formulae

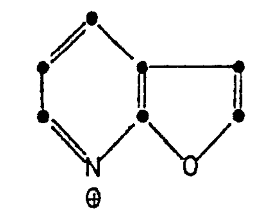 , 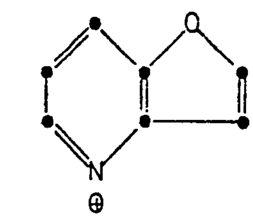

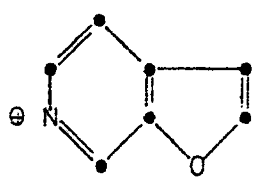 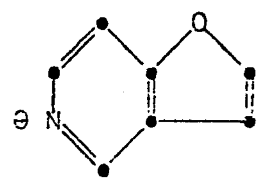 ,

where in the above formulae either or both of the hetero rings is optionally substituted by one or two substituents independently selected from $C_1$-$C_4$ alkyl, halogen, trifluoromethyl, carboxy, carbamoyl, $-SO_3H$, hydroxy, $C_1$-$C_4$ alkoxy, amino, mono $C_1$-$C_4$ alkylamino, di($C_1$-$C_4$) alkylamino, $C_{2-4}$ alkanoylamino, aminosulphonyl, cyano, $C_1$-$C_4$ alkoxycarbonyl or a group of formula

$$-\overset{O}{\overset{\|}{C}}-NR_5-OR_6$$

where $R_5$ and $R_6$ are independently hydrogen or $C_1$-$C_4$ alkyl; or a pharmaceutically-acceptable salt thereof, which comprises:

(a) condensing a compound of formula:

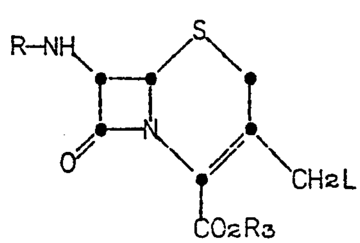 (II)

where L is a leaving group and $R_3$ is hydrogen or an ester protecting group; with a thieno or furopyridine of formula $R_1$, followed, in the case where $R_3$ is an ester protecting group, by removal of that group and any protecting groups which may be present in the R-substituent;

(b) acylating a compound of formula

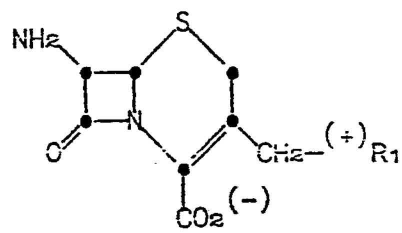

(III)

, where $R_1$ is as defined above, or a salt or 4'-ester ($R_3$) thereof, with an active carboxy derivative of an acid of formula

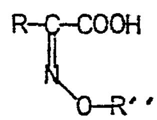

followed, in the case where the compound of formula (III) is in the form of an ester, by removal of the $R_3$ group, together with any amino protecting groups which may be present in R-substituent.

2. A process according to claim 1 in which R is

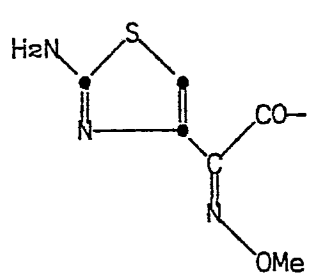

in which the oxime has a syn-configuration.

3. A process according to claim 1 or 2, wherein either or both of the thieno- or furopyridinium rings is optionally substituted by one or two $C_1-C_4$ alkyl, fluoro, chloro, bromo, carboxy, carbamoyl or $C_1-C_4$ alkoxycarbonyl groups.

4. A process according to claim 3, wherein either or both of the thieno or furopyridinium rings is substituted by carboxy.

5. A process according to claim 4 for preparing syn-7-[2-(2-aminothiazol-4-yl)-2-methoxyiminoacetamido]-3-(2-carboxythieno[3,2-c]pyridinium-5-ylmethyl)-3-cephem-4-carboxylate.

6. A process according to claim 1 or 2, wherein $^{(+)}R_1$ is an unsubstituted thienopyridinium group.

7. A cephalosporin derivative of formula (I), or a pharmaceutically-acceptable salt thereof, whenever prepared by a process according to any one of claims 1 to 6.